# EUROPEAN PATENT SPECIFICATION

(11) **EP 3 465 504 B1**
(45) Date of publication and mention of the grant of the patent: **09.12.2026**
(21) Application number: 17807056.1
(22) Date of filing: 02.06.2017
(51) Int. Cl.: G16B 40/00, G06F 17/18, C12Q 1/68

(54) **METHOD FOR DETECTING A TARGET ANALYTE IN A SAMPLE USING A SIGNAL CHANGE-AMOUNT DATA SET**
VERFAHREN ZUM NACHWEIS EINES ZIELANALYTEN IN EINER PROBE UNTER VERWENDUNG EINES DATENSATZES MIT SIGNALWECHSELMENGE
PROCÉDÉ DE DÉTECTION D'UN ANALYTE CIBLE DANS UN ÉCHANTILLON À L'AIDE D'UN ENSEMBLE DE DONNÉES DE QUANTITÉ DE CHANGEMENT DE SIGNAL

(30) Priority: 02.06.2016 KR 20160068932
(43) Date of publication of application: 10.04.2019
(73) Proprietor: Seegene, Inc., Seoul 05548 (KR)
(72) Inventor: CHUN, Jong Yoon, Seoul 06075 (KR); LEE, Young Jo, Seoul 05507 (KR); LEE, Han Bit, Seoul 05549 (KR)
(74) Representative: Kilger, Ute
(86) International application number: PCT/KR2017/005790
(87) International publication number: WO 2017/209563

(56) References cited:
- WO-A2-2014/186526
- KR-A- 20160 039 529
- US-A1- 2008 154 511
- US-A1- 2012 072 120
- US-A1- 2014 127 793
- US-A1- 2015 186 598
- US-A1- 2015 186 598
- US-A1- 2015 232 916
- US-A1- 2016 040 222

## Description

### FIELD OF THE INVENTION

The present invention relates to a method for detecting a target analyte in a sample using a signal change-amount data set.

### BACKGROUND OF THE INVENTION

A polymerase chain reaction (hereinafter referred to as "PCR") which is most widely used for the nucleic acid amplification includes repeated cycles of denaturation of double-stranded DNA, followed by oligonucleotide primer annealing to the DNA template, and primer extension by a DNA polymerase (Mullis et al., U. S. Pat. Nos. 4,683,195, 4,683,202 and 4,800,159; Saiki et al., (1985) Science 230, 1350-1354).

A real-time polymerase chain reaction is one of PCR-based technologies for detecting a target nucleic acid molecule in a sample in a real-time manner. For detecting a specific target analyte, the real-time PCR uses a signal-generating means for generating a fluorescence signal being detectable in a proportional manner with the amount of the target molecule. The generation of fluorescence signals may be accomplished by using either intercalators generating signals when intercalated between double-stranded DNA or oligonucleotides carrying fluorescent reporter and quencher molecules. The fluorescence signals whose intensities are proportional with the amount of the target molecule are detected at each amplification cycle and plotted against amplification cycles, thereby obtaining an amplification curve or amplification profile curve.

In general, an amplification curve of the real-time PCR may be classified into a baseline region, an exponential phase, linear phase and a plateau phase. The exponential phase shows increase in fluorescent signals in proportional to increase of amplification products. In the linear phase, the increase in fluorescent signals is substantially reduced and behaves in a substantially linear manner and the plateau phase refers to a region in which there is little increase in fluorescent signals due to saturation of both PCR amplicon and fluorescent signal levels.

The baseline region refers to a region in which there is little change in fluorescent signal during initial cycle of PCR. In the baseline region, the level of PCR amplicon is not sufficient to be detectable and therefore signals detected in this region may be due to background signal involving fluorescent signals from reaction reagents and measurement device.

Problems such as generation of noise, distortion or fluctuation of baseline occur due to inter- and intra-instrument variations in signal level, variation of experimental conditions such as variations in annealing temperature, and generation of bubbles in a reaction mixture. Such noise or baseline distortion or fluctuation may result in false positive or false negative results in the analysis of the target analyte. For an accurate and reproducible analysis of a data set for a target analyte, a baselining process removing a background signal from a data set or an amplification curve and a process of correcting an abnormal signal are needed.

According to conventional methods for target analysis, the baselining process is performed by determining a baseline region and a baseline of data set for each sample and then removing a background signal of the baseline region. Particularly, the baseline region determination is performed through determination of a start point and an end point of the baseline region. It is therefore important to accurately determine the start point and end point of the baseline region.

Various methods have been developed for the determination of a baseline region. Woo *et al.* discloses a method for determining a baseline region using a lower bound of an amplification region (US Publication No. 2007/0192040). Lerner et al. discloses a method for determining a baseline region by differentiating an amplification curve and then setting a start point of the first differentiation peak that have a signal value more than threshold value as an end point of the baseline region (US Pat. No. 7,720,611).

However, these prior art may have some limitations or shortcomings. According to conventional methods, a noise or abnormal signal generated in the baseline region may affect the baseline determination. In particular, when a start point or end point of baseline region is determined by a noise or an abnormal signal, a baselining process using a linear fit function of baseline is greatly affected by the noise or abnormal signal.

In order to prevent such an error, various methods have been proposed to eliminate or avoid the noise or abnormal signal by setting up a certain criterion and determining a signal corresponding to the criterion as a noise or an abnormal one.

However, despite these efforts, errors in detection of target analyte caused by false baseline region setting are occurring frequently. Even more problematic is that the more an algorism for the baseline region setting is complicated and strict, the more a normal signal is mistakenly determined as a noise or an abnormal signal, so that the probability of generating a new error in determination of a baseline region is increased.

Moreover, in conventional methods, a data set is processed for detecting data points that need to be corrected followed by detecting data points having noise signal using the processed data set, and then a signal values of the data points that need to be corrected in original data set are altered. In conclusion, detection and correction of a noise or an abnormal signal is performed in different data set, respectively. Furthermore, in case of an error that continuously affects the entire subsequent cycle (*e*.*g*., jump error), each signal value of entire subsequent cycle must be corrected in conventional method.

Accordingly, there are strong needs in the art to develop novel approaches for calibrating a data set without complicate establishment of baseline region.

Throughout this application, various patents and publications are referenced and citations are provided in parentheses. The disclosure of these patents and publications in their entirety are hereby incorporated by references into this application in order to more fully describe this invention and the state of the art to which this invention pertains.

Document US 2015/232916 A1 discloses methods and devices for simultaneously amplifying a plurality of sample wells for a predetermined amount of amplification, detecting whether amplification has occurred in a first set of the wells, amplifying for an additional amount of amplification and detecting whether amplification has occurred in a second set of the wells. Methods are also provided for analyzing a target nucleic acid sequence using melt curves that were generated in a plurality of amplification cycles.

The invention disclosed in document US 2014/127793 A1, in various embodiments, provides novel methods for discriminating two or more different target nucleic acids. In certain embodiments the methods comprise providing data amplification reactions comprising reagents to amplify two or more different target nucleic acids where the data comprise signals comprising an amplitude measurement representing the degree of amplification of each target nucleic acid in the amplification reaction and the time point in the amplification reaction at which the amplitude is measured; determining an efficiency related transform of the data, determining an efficiency related value for each target nucleic acid that is the maximum magnitude of the efficiency related transform; and outputting the efficiency related values in the amplification reaction for each target nucleic acid, where the relative amplitudes of the efficiency related values for each target nucleic acid is an indicator of the presence of each of said nucleic acids in said sample.

The document KR 2016 0039529 A discloses a method for correcting an amplification curve obtained from an amplification reaction performed in presence of a signal generating means. More particularly, the method for correcting an amplification curve comprises the following steps: (a) obtaining a raw data set of amplification cycles and signals measured from the cycles by a signal generating means from an amplification reaction; (b) determining a baseline region by determining a start cycle and an end cycle of a baseline from the raw data set; (c) computing an optimum linear function in the baseline region from at least two data in the determined baseline region; and (d) obtaining a corrected data set by subtracting function values calculated by the optimum linear function in the baseline region from measured signals in the raw data set from step (a). Further, the present invention relates to a recording means of the method for correcting an amplification curve, a computer program thereof, and a computer device. The method for correcting an amplification curve in the present invention can compute objective and accurate results of an experiment by objectively setting a baseline region for each sample.

In document US 2015/186598 A1 systems, methods, and apparatuses for detecting and potentially invalidating or correcting jump errors in data from growth processes are disclosed. A jump error can be identified by determining a second derivative of the data set, and identifying two consecutive cycles with opposite signs in the second derivative. Once a jump error has been detected, the data set can be invalidated or corrected based on various criteria. Whether to invalidate or correct can be based on an absolute jump height, a relative jump height (e.g., relative to the net growth or relative to the baseline), an absolute location (cycle number) of the jump, or a relative location. In one implementation, the jump can be corrected by subtracting a jump height from points subsequent to the jump or by adding the jump height to points prior to the jump.

### SUMMARY OF THE INVENTION

The present invention is defined by the appended claims.

### SUMMARY OF RELATED DISCLOSURE

The present inventor have made intensive researches to develop novel approaches for processing a data set of a sample which enables us to more efficiently and accurately analyze a target analyte in a sample. As a result, we have found that a processed data set suitable for a sample analysis can be obtained by providing a signal change-amount data set by obtaining a signal change amount at each cycle using a signal values of a data set and obtaining a reconstructed data set by obtaining a cumulated value at each cycle using the signal change amounts of the signal change-amount data set.

Accordingly, it is an object of this invention to provide a method for detecting a target analyte in a sample.

It is another object of this invention to provide a method for reconstructing a data set.

It is another object of this invention to provide a method for smoothing a data set.

It is still another object of this invention to provide a computer readable storage medium containing instructions to configure a processor to perform a method for detecting a target analyte in a sample.

It is still another object of this invention to provide a computer readable storage medium containing instructions to configure a processor to perform a method for reconstructing a data set.

It is still another object of this invention to provide a computer readable storage medium containing instructions to configure a processor to perform a method for smoothing a data set.

Other objects and advantages of the present invention will become apparent from the detailed description to follow taken in conjugation with the appended claims and drawings.

### BRIEF DESCRIPTION OF THE DRAWINGS

Fig. 1 represents a flow diagram illustrating an embodiment of the present method for detecting a target analyte in a sample comprising the steps of providing a signal change-amount data set and providing a reconstructed data set.

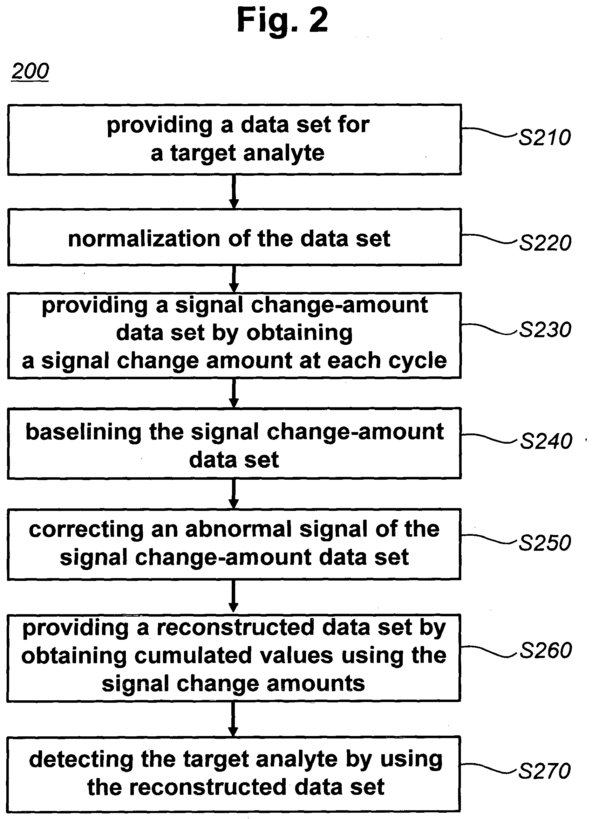

Fig. 2 represents a flow diagram illustrating an embodiment of the present method detecting a target analyte in a sample comprising the steps of (i) normalization of a data set; (ii) a baselining of a signal change-amount data set and (iii) amending an abnormal signal of the signal change-amount data set.
Fig. 3 represents plots of the three raw data sets used in an embodiment of the present method.
Fig. 4 represents plots of data set 1 obtained in each steps of process according to an embodiment of the present invention.
Fig. 5A represents plots of data sets 2 obtained in each steps of process according to an embodiment of the present invention with or without further applying a baselining step.
Fig. 5B represents plots of data set 3 obtained in each steps of process according to an embodiment of the present invention including a baselining step.
Fig. 6A shows that the individual reconstructed data sets have different signal values at the same cycle when the data sets were obtained by using different cumulation-starting cycles. However, the respective subtracted data sets have the same signal value at the same cycle regardless of the difference of the "cumulation-starting cycle".
Fig. 6B shows that the individual reconstructed data sets have different signal values at the same cycle when the data sets were obtained by using different cumulation-starting values. However, the respective subtracted data sets have the same signal value at the same cycle regardless of the difference of the "cumulation-starting value".
Fig. 7 shows that the signal change-amount data set could be obtained by various methods for calculating the signal change amount from the raw data set.
Fig. 8 represents a flow diagram illustrating an embodiment of the present method for detecting a target analyte in a sample comprising the steps of providing a signal change-amount data set; amending an abnormal signal value of the signal change-amount data set and providing a reconstructed data set.
Fig. 9 represents plots of the three raw data sets used in an embodiment of the present method including a step of amending an abnormal signal value.
Fig. 10 represents a process of detecting and removing an abnormal signal value using a signal change-amount data set.
Fig. 11A represents a result of comparing plots of three reconstructed data sets with/without amendment for removing an abnormal signal value.
Fig. 11B represents a result of comparing plots of reconstructed data sets with/without applying a noise correction step of the present invention.

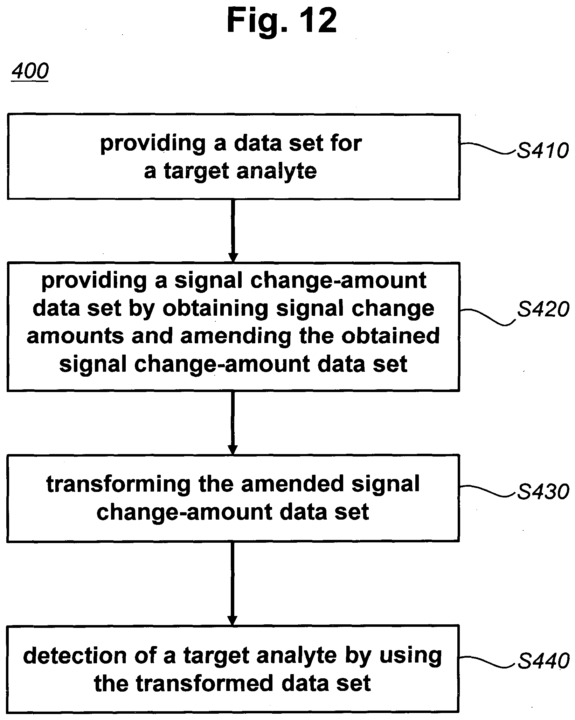

Fig. 12 represents a flow diagram illustrating an embodiment of the present method for detecting a target analyte in a sample comprising the steps of amending a signal change-amount data set and transforming the amended signal change-amount data set.
Fig. 13 represents the plots of the three raw data sets used in an embodiment of the present method for detecting a target analyte in a sample comprising the steps of amending a signal change-amount data set and transforming the amended signal change-amount data set.
Fig. 14 represents a result of comparing the plots of 2^{nd} order signal change-amount data set obtained by transforming a signal change-amount data set with/without applying an amendment step of the present invention.
Fig. 15 represents a flow diagram illustrating an embodiment of the present method for smoothing a data set.
Fig. 16A represents plots of reconstructed data set 1 for each repetition.
Fig. 16B represents the background region of the plots of the reconstructed data set 1 for each repetition.

### DETAILED DESCRIPTION OF THIS INVENTION

### I. Method for Detecting a Target Analyte in a Sample Using a Reconstructed Data Set

In one aspect of this invention, there is provided a method for detecting a target analyte in a sample comprising:
(a) providing a data set for a target analyte; wherein the data set is obtained from a signal-generating process for the target analyte using a signal-generating means; wherein the data set comprises a plurality of data points, each data point comprising a cycle and a signal value at the cycle;
(b) providing a signal change-amount data set by obtaining a signal change amount at each cycle using the signal values of the data set; wherein the signal change-amount data set comprises a plurality of data points comprising cycles and signal change amounts in each cycle;
(c) providing a reconstructed data set by obtaining a cumulated value at each cycle using the signal change amounts of the signal change-amount data set; wherein the reconstructed data set comprises a plurality of data points comprising cycles and cumulated values at the cycles; and
(d) detecting the target analyte in the sample by using the reconstructed data set.

The present inventor have made intensive researches to develop novel approaches for processing a data set of a sample which enables us to more efficiently and accurately analyze a target analyte in a sample. As a result, we have found that a processed data set suitable for a sample analysis can be obtained by (i) providing a signal change-amount data set by obtaining a signal change amount at each cycle using a signal values of a data set and (ii) providing a reconstructed data set by obtaining a cumulated value at each cycle using the signal change amounts of the signal change-amount data set.

In the method of the present disclosure, a data set for a target analyte is converted to a signal change-amount data set that comprises a plurality of data points comprising cycles and signal change amounts at the cycles, and then the signal change-amount data set is reconstructed to give a reconstructed data set that comprises a plurality of data points of cycles and cumulated values at the cycles.

Through such a conversion and reconstruction process, a data set may be processed to be suitable for the detection of a target analyte. In addition, the modifications (e.g., correction or removal of abnormal signals or noise signals) having been conducted on the signal change-amount data set may be reflected in the final reconstructed data set through the reconstruction process. In this way, it possible to process a data set for detecting a target analyte without directly correcting a signal value of the data set.

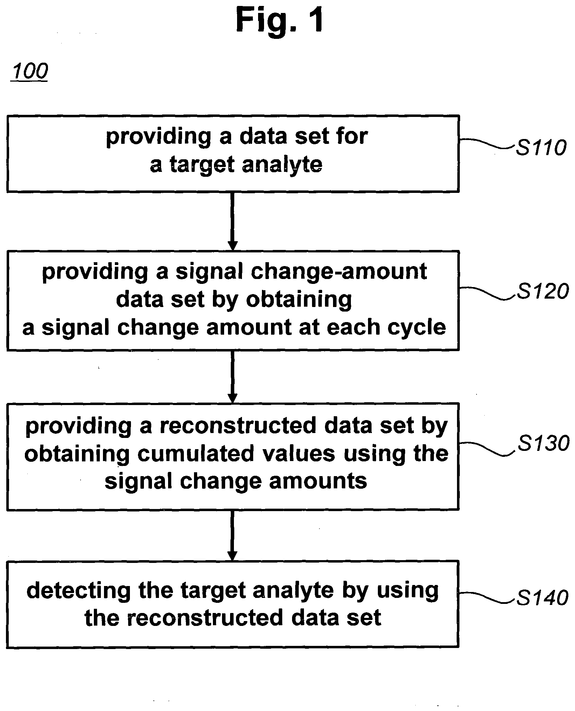

Fig. 1 represents a flow diagram illustrating an embodiment of the present method of detecting a target analyte in a sample using the reconstructed data set for the target analyte.

The present invention will be described in more detail as follows:

### Step (a): Providing a data set for a target analyte (S110)

According to the present method, a data set for a target analyte is provided. The data set is obtained from a signal-generating process for a target analyte using a signal-generating means, and the data set comprises a plurality of data points comprising cycles and signal values of the signal-generating process.

The term used herein "target analyte" may include various materials (*e*.*g*., biological materials and non-biological materials such as chemicals). Particularly, the target analyte may include biological materials such as nucleic acid molecules (*e*.*g*., DNA and RNA), proteins, peptides, carbohydrates, lipids, amino acids, biological chemicals, hormones, antibodies, antigens, metabolites and cells. More particularly, the target analyte may include nucleic acid molecules. According to an embodiment, the target analyte may be a target nucleic acid molecule.

The term used herein "sample" may include biological samples (*e.g*., cell, tissue and fluid from a biological source) and non-biological samples (*e*.*g*., food, water and soil). The biological samples may include virus, bacteria, tissue, cell, blood (*e*.*g*., whole blood, plasma and serum), lymph, bone marrow aspirate, saliva, sputum, swab, aspiration, milk, urine, stool, vitreous humour, sperm, brain fluid, cerebrospinal fluid, joint fluid, fluid of thymus gland, bronchoalveolar lavage, ascites and amnion fluid. When a target analyte is a target nucleic acid molecule, the sample is subjected to a nucleic acid extraction process. When the extracted nucleic acid is RNA, reverse transcription process is performed additionally to synthesize cDNA from the extracted RNA(Joseph Sambrook, et al., Molecular Cloning, A Laboratory Manual, Cold Spring Harbor Laboratory Press, Cold Spring Harbor, N.Y.(2001)).

According to an embodiment, the data set is obtained from a signal-generating process for the target analyte using a signal-generating means. According to an embodiment, the method may further comprise the step of performing the signal-generating process to obtain a data set of the target analyte in the sample.

The term used herein "signal-generating process" refers to any process capable of generating signals in a dependent manner on a property of a target analyte in a sample, wherein the property may be, for instances, activity, amount or presence (or absence) of the target analyte, in particular the presence of (or the absence of) an analyte in a sample. According to an embodiment, the signal-generating process generates signals in a dependent manner on the presence of the target analyte in the sample.

Such signal-generating process may include biological and chemical processes. The biological processes may include genetic analysis processes such as PCR, real-time PCR, microarray and invader assay, immunoassay processes and bacteria growth analysis. According to an embodiment, the signal-generating process includes genetic analysis processes. The chemical processes may include a chemical analysis comprising production, change or decomposition of chemical materials. According to an embodiment, the signal-generating process may be a PCR or a real-time PCR.

According to an embodiment, the signal-generating process may be a process of amplifying the signal values. The term used herein "amplification" or "amplification reaction" refers to a reaction for increasing or decreasing signals. According to an embodiment of this invention, the amplification reaction refers to an increase (or amplification) of a signal generated depending on the presence of the target analyte by using the signal-generating means. The amplification reaction is accompanied with or without an amplification of the target analyte (e.g., nucleic acid molecule). Therefore, according to an embodiment of this invention , the signal-generating process is performed with or without an amplification of the target nucleic acid molecule. More particularly, the amplification reaction of present invention refers to a signal amplification reaction performed with an amplification of the target analyte.

The signal-generating process may be accompanied with a signal change. The term "signal" as used herein refers to a measurable output.

The signal change may serve as an indicator indicating qualitatively or quantitatively the property, in particular the presence or absence of a target analyte. Examples of useful indicators include fluorescence intensity, luminescence intensity, chemiluminescence intensity, bioluminescence intensity, phosphorescence intensity, charge transfer, voltage, current, power, energy, temperature, viscosity, light scatter, radioactive intensity, reflectivity, transmittance and absorbance. The most widely used indicator is fluorescence intensity. The signal change may include a signal decrease as well as a signal increase. According to an embodiment, the signal-generating process is a process of amplifying the signal values.

The term used herein "signal-generating means" refers to any material used in the generation of a signal indicating a property, more specifically the presence or absence of the target analyte which is intended to be analyzed.

A wide variety of the signal-generating means have been known to one of skill in the art. Examples of the signal-generating means may include oligonucleotides, labels and enzymes. The signal-generating means include both labels *per se* and oligonucleotides with labels. The labels may include a fluorescent label, a luminescent label, a chemiluminescent label, an electrochemical label and a metal label. The label *per se* like an intercalating dye may serve as signal-generating means. Alternatively, a single label or an interactive dual label containing a donor molecule and an acceptor molecule may be used as signal-generating means in the form of linkage to at least one oligonucleotide. The signal-generating means may comprise additional components for generating signals such as nucleolytic enzymes (*e*.*g*., 5'-nucleases and 3'-nucleases).

The signal-generating means may comprise (1) means that generates signals in a manner dependent on formation of a dimer; (2) means that generates signals by formation of a dimer in a manner dependent on cleavage of mediation oligonucleotide specifically hybridized to a target analyte; and (3) means that generates signals by a cleavage of a detection-oligonucleotide.

Where the present method is applied to determination of the presence or absence of a target nucleic acid molecule, the signal-generating process may be performed in accordance with a multitude of methods known to one of skill in the art. The methods include TaqMan^{™} probe method (U.S. Pat. No. 5,210,015), Molecular Beacon method (Tyagi et al., Nature Biotechnology, 14 (3):303(1996)), Scorpion method (Whitcombe et al., Nature Biotechnology 17:804-807(1999)), Sunrise or Amplifluor method (Nazarenko et al., Nucleic Acids Research, 25(12):2516-2521(1997), and U.S. Pat. No. 6,117,635), Lux method (U.S. Pat. No. 7,537,886), CPT (Duck P, et al., Biotechniques, 9:142-148(1990)), LNA method (U.S. Pat. No. 6,977,295), Plexor method (Sherrill CB, et al., Journal of the American Chemical Society, 126:4550-4556(2004)), Hybeacons^{™} (D. J. French, et al., Molecular and Cellular Probes (2001) 13, 363-374 and U.S. Pat. No. 7,348,141), Dual-labeled, self-quenched probe (US 5,876,930), Hybridization probe (Bernard PS, et al., Clin Chem 2000, 46, 147-148), PTOCE (PTO cleavage and extension) method (WO 2012/096523), PCE-SH (PTO Cleavage and Extension-Dependent Signaling Oligonucleotide Hybridization) method (WO 2013/115442) and PCE-NH (PTO Cleavage and Extension-Dependent Non-Hybridization) method (PCT/KR2013/012312) and CER method (WO 2011/037306). Therefore, in the present invention, the amplification reaction can be performed by the above-described signal-generating processes.

The term used herein "cycle" refers to a unit of changes of conditions or a unit of a repetition of the changes of conditions in a plurality of measurements accompanied with changes of conditions. For example, the changes of conditions or the repetition of the changes of conditions include changes or repetition of changes in temperature, reaction time, reaction number, concentration, pH and/or replication number of a measured subject (*e*.*g*., target nucleic acid molecule). Therefore, the cycle may include a condition (*e*.*g*., temperature or concentration) change cycle, a time or a process cycle, a unit operation cycle and a reproductive cycle. A cycle number represents the number of repetition of the cycle. In this document, the terms "cycle" and "cycle number" are used interchangeably.

For example, when enzyme kinetics is investigated, the reaction rate of an enzyme is measured several times as the concentration of a substrate is increased regularly. In this reaction, the increase in the substrate concentration may correspond to the changes of the conditions and the increasing unit of the substrate concentration may be corresponding to a cycle. For another example, when an isothermal amplification of nucleic acid is performed, the signals of a single sample are measured multiple times with a regular interval of times under isothermal conditions. In this reaction, the reaction time may correspond to the changes of conditions and a unit of the reaction time may correspond to a cycle. According to another embodiment, as one of methods for detecting a target analyte through a nucleic acid amplification reaction, a plurality of fluorescence signals generated from the probes hybridized to the target analyte are measured with a regular change of the temperature in the reaction. In this reaction, the change of the temperature may correspond to the changes of conditions and the temperature may correspond to a cycle.

Particularly, when repeating a series of reactions or repeating a reaction with a time interval, the term "cycle" refers to a unit of the repetition. For example, in a polymerase chain reaction (PCR), a cycle refers to a reaction unit comprising denaturation of a target nucleic acid molecule, annealing (hybridization) between the target nucleic acid molecule and primers and primer extension. The increases in the repetition of reactions may correspond to the changes of conditions and a unit of the repetition may correspond to a cycle.

According to an embodiment, where the target nucleic acid molecule is present in a sample, values (*e*.*g*., intensities) of signals measured are increased or decreased upon increasing cycles of an amplification reaction. According to an embodiment, the amplification reaction to amplify signals indicative of the presence of the target nucleic acid molecule may be performed in such a manner that signals are amplified simultaneously with the amplification of the target nucleic acid molecule (*e*.*g*., real-time PCR). Alternatively, the amplification reaction may be performed in such a manner that signals are amplified with no amplification of the target nucleic acid molecule [*e*.*g*., CPT method (Duck P, et al., Biotechniques, 9:142-148 (1990)), Invader assay (U.S. Pat. Nos. 6,358,691 and 6,194,149)].

The target analyte may be amplified by various methods. For example, a multitude of methods have been known for amplification of a target nucleic acid molecule, including, but not limited to, PCR (polymerase chain reaction), LCR (ligase chain reaction, see U.S. Pat. Nos. 4683195 and 4683202; A Guide to Methods and Applications (Innis et al., eds, 1990); Wiedmann M, et al., "Ligase chain reaction (LCR)- overview and applications." PCR Methods and Applications 1994 Feb;3(4):S51-64), GLCR (gap filling LCR, see WO 90/01069, EP 439182 and WO 93/00447), Q-beta (Q-beta replicase amplification, see Cahill P, et al., Clin Chem., 37(9):1482-5(1991), U.S. Pat. No. 5556751), SDA (strand displacement amplification, see G T Walker et al., Nucleic Acids Res. 20(7):1691-1696(1992), EP 497272), NASBA (nucleic acid sequence-based amplification, see Compton, J. Nature 350(6313):91-2(1991)), TMA (Transcription-Mediated Amplification, see Hofmann WP et al., J Clin Virol. 32(4):289-93(2005); U.S. Pat. No. 5888779) or RCA (Rolling Circle Amplification, see Hutchison C.A. et al., Proc. Natl Acad. Sci. USA. 102:17332-17336(2005)).

According to an embodiment, the label used for the signal-generating means may comprise a fluorescence, more particularly, a fluorescent single label or an interactive dual label comprising donor molecule and acceptor molecule (*e*.*g*., an interactive dual label containing a fluorescent reporter molecule and a quencher molecule).

According to an embodiment, the data set for a target analyte may be a data set representing a result of an amplification reaction for the target analyte.

According to an embodiment, the amplification reaction used in the present invention may amplify signals simultaneously with amplification of the target analyte, particularly the target nucleic acid molecule. According to an embodiment, the amplification reaction is performed in accordance with a PCR or a real-time PCR. According to an embodiment, the amplification reaction may be a amplification reaction of a nucleic acid molecule.

The data set obtained from a signal-generating process comprises a plurality of data points comprising cycles of the signal-generating process and signal values at the cycles.

The term used herein "values of signals" or "signal values" means either values of signals actually measured at the cycles of the signal-generating process (*e*.*g*., actual value of fluorescence intensity processed by amplification reaction) or their modifications. The modifications may include mathematically processed values of measured signal values (*e*.*g*., intensities). Examples of mathematically processed values of measured signal values may include logarithmic values and derivatives of measured signal values. The derivatives of measured signal values may include multi-derivatives.

The term used herein "data point" means a coordinate value comprising a cycle and a value of a signal at the cycle. The term used herein "data" means any information comprised in data set. For example, each of cycles and signal values of an amplification reaction may be data. The data points obtained from a signal-generating process, particularly from an amplification reaction may be plotted with coordinate values in a rectangular coordinate system. In the rectangular coordinate system, the X-axis represents cycles of the amplification reaction and the Y-axis represents signal values measured at each cycles or modifications of the signal values.

The term used herein "data set" refers to a set of data points. The data set may include a raw data set which is a set of data points obtained directly from the signal-generating process (*e*.*g*., an amplification reaction) using a signal-generating means. Alternatively, the data set may be a modified data set which is obtained by a modification of the data set including a set of data points obtained directly from the signal-generating process. The data set may include an entire or a partial set of data points obtained from the signal-generating process or modified data points thereof. The data set comprises a plurality of data points. The data set may comprise at least 2 data points. The number of data points may be at least 2, 3, 4, 5, 10 or 20. The data set may comprise not more than 1000 data points. The number of data points in a data set may be not more than 1000, 500, 300, 200, 100, 90, 80, 70 or 60. The data set may comprise 3-1000 data points.

The number of data points in a data set may be 3-1000, 10-500, 1-100, 20-100, 20-80, 20-70 or 20-60. According to an embodiment, the data set may comprise 20-60 data points.

The data set of the present invention may be obtained by processing a plurality of data sets. Where analysis of a plurality of target analyte materials is performed in one reaction vessel, the data sets for each of the target analyte materials may be obtained through the processing of raw data sets obtained from the reactions performed in the one reaction vessel. For example, data sets for a plurality of target analyte materials obtained in one reaction vessel may be obtained by processing a plurality of data sets obtained from signals measured at different temperatures. The data set may be plotted and whereby an amplification curve may be obtained. The fluorescent signals whose intensities are proportional with the amount of the target molecule are detected at each amplification cycle and plotted against amplification cycles, thereby obtaining an amplification curve or amplification profile curve. According to an embodiment, an amplification curve may be obtained by an amplification reaction for a target analyte (particularly, a nucleic acid molecule).

According to an embodiment, the data set may be a mathematically processed data set of the raw data set. In particular, the data set may be a baseline subtracted data set for removing a background signal value from the raw data set. The baseline subtracted data set may be obtained by methods well known in the art (*e*.*g*., US 8,560,240).

According to an embodiment, the data set of the step (a) may be a raw data set, a mathematically modified data set of the raw data set, a normalized data set of the raw data set or a normalized data set of the modified data set of the raw data set.

The term "raw data set" as used herein refers to a set of data points (including cycle numbers and signal values) obtained directly from a signal-generating process. The raw data set means a set of non-processed data points which are initially received from a device for performing a signal-generating process such as a real-time PCR (e.g., thermocycler, PCR machine or DNA amplifier). In an embodiment of the present invention, the raw data set may include a raw data set understood conventionally by one skilled in the art. In an embodiment of the present invention, the raw data set may include a data set prior to processing. In another embodiment, the raw data set may include a dataset which is the basis for the mathematically processed data sets as described herein. In an embodiment of the present invention, the raw data set may include a data set not subtracted by a baseline (no baseline subtraction data set).

The term used herein "normalization" refers to a process of reducing or eliminating a signal variation of a data set obtained from a signal-generating process. The term used herein "calibration" or "adjustment" refers to a correction of a data set, particularly a correction of a signal value of a data set, suitable for the aim of analysis. The normalization is one aspect of the calibration.

According to an embodiment, the normalized data set may be provided by a method comprising the steps of:
(i) providing a normalization coefficient for calibrating the raw data set or the mathematically modified data set of a raw data set; wherein the normalization coefficient is provided by using a reference value, a reference cycle and the data set; wherein the reference cycle is selected from the cycles of the data set; wherein the reference value is an arbitrarily determined value; wherein the normalization coefficient is provided by defining a relationship between the reference value and a signal value at a cycle of the data set corresponding to the reference cycle; and
(ii) providing a normalized data set by obtaining calibrated signal values by applying the normalization coefficient to the signal values of the data set.

The reference cycle is a cycle selected for determining a specific signal value used for providing a normalization coefficient with a reference value. The reference cycle used for providing a normalization coefficient may be selected arbitrarily from cycles of the data set. The reference cycle may encompass a reference temperature, a reference concentration or a reference time depending on the meaning of the cycle.

According to an embodiment, the reference cycle may be selected from the cycles in a background region.

The background region refers to an early stage of a signal-generating process before amplification of signal is sufficiently detected. A background region is a region in which only a background signal is generated and the signal due to a target analyte rarely occurs. The background signal is a signal generated by an analytical system itself, or by the signal-generating means itself not involved in the target analyte, not by a target analyte in a sample.

Specifically, the reference cycle may be determined from the cycles 1-30, 2-30, 2-20, 2-15, 2-10, 2-8, 3-30, 3-20, 3-15, 3-10, 3-9, 3-8, 4-8, or 5-8 in the background region.

A reference value is a value used for providing a normalization coefficient. A reference value of the present invention refers to an arbitrary value that is applied to a reference cycle for the calibrations of signal values of a data set. A reference value may be an arbitrarily determined value. Preferably, the reference value may be an arbitrarily determined value from a real number except zero. Preferably, a reference value may be the same-typed value as the values of a data set to be calibrated and may have the same unit or dimension as the data set to be calibrated.

When the normalization coefficient is provided by a reference value and a signal value at the reference cycle-corresponding cycle of the data set, the normalization coefficient may be provided by a ratio of the signal values at the reference cycle-corresponding cycle of the data set to the reference value.

### Step (b): Providing a signal change-amount data set by obtaining signal chance amounts (S120)

A signal change-amount data set may be provided by obtaining signal change values at each cycle using the signal values of the data set

The signal change-amount data set represents a signal change amount of each data points in the data set. The signal change-amount data set comprises a plurality of data points comprising cycles and signal change amounts at each cycle.

The signal change-amount data set may encompass a change-value data set and a change-ratio data set. The signal change-amount may encompass a signal change-value and a signal change-ratio.

The signal change amount may be obtained by a method known in the art, for example the method may include a differentiation method, a difference method, a ratio method and linear regression analysis method but not be limited to these methods.

According to an embodiment, the signal change amount at each cycle may be selected from the group consisting of a derivative value of the signal values at each cycle; a difference in the signal values at each cycle; a ratio of the signal values at each cycle; and a slope value obtained by performing a linear regression analysis at each cycle.

According to a differentiation method, the signal change amount may be obtained by setting up a function optimally fitted to a raw data set, obtaining a derivative function from the function, and then obtaining a signal change amount at each cycle using the derivative function.

According to a difference method, a signal change amount at a cycle (target cycle; Cₙ) is obtained by calculating a difference of signal value between the two cycles (reference cycle 1 and 2).

In an embodiment, one of the reference cycles may be the target cycle (Cₙ) and the other reference cycle may be an immediately preceding cycle (Cₙ₋₁) of the target cycle. In this case, a signal change amount at Cₙ may be obtained by subtracting a signal value at Cₙ₋₁ from a signal value at Cₙ. Where one of the reference cycles is designated as Cₙ, the other reference cycle may be Cₙ₋₁, Cₙ₋₂, Cₙ₋₃, Cₙ₊₁, Cₙ₊₂ or Cₙ₊₃.

In another embodiment, none of the two reference cycles may be designated as Cₙ. For example, two reference cycles that are used for obtaining a signal change value at a target cycle (Cₙ) may be (Cₙ₋₁ and Cₙ₊₁), (Cₙ₋₂ and Cₙ₊₂) or (Cₙ₋₃ and Cₙ₊₃).

According to an embodiment, the signal change amount at a target cycle may be a value that is obtained by dividing a difference of signal values between the two reference cycles by a difference of cycle numbers between the two reference cycles.

According to a ratio method, a signal change amount at a cycle (target cycle; Cₙ) is obtained by calculating a ratio of signal values between the two cycles (reference cycle 1 and 2).

In an embodiment, one of the reference cycles may be the target cycle (Cₙ) and the other reference cycle may be an immediately preceding cycle (Cₙ₋₁) of the target cycle. In this case, a signal change value at Cₙ may be obtained by dividing a signal value at Cₙ by a signal value at Cₙ₋₁.

Alternatively, the signal change amount may be provided by a linear regression analysis or a least mean square (LMS) method. The LMS method is the simplest and most commonly applied embodiment of a linear regression analysis.

According to a linear regression analysis method, a fitting function at a cycle (target cycle; Cₙ) is provided by linear regression analysis using a data point of a certain cycle and at least one data point of the cycles before and/or after the certain cycle and then a slope of the fitting function (an amount of change in signal value as increase of a cycle number) is assigned as a signal change amount.

The number of the data points used for obtaining a fitting function may be two or more. For example, the number of the data points may be not more than 2, 3, 4, 5, 6, 7, 8, 9, 10, 11, 12, 13, 14, 15, 16, 17, 18, 19, 20, 21. Particularly, the number of the data points may be 2-3, 2-15, 3-21, 3-11, 3-9, 3-7, 3-5 or 5-7 but not limited to these ranges.

The below descriptions illustrate a least square method as a representative of a linear regression analysis but the scope of the present invention as set forth in the appended claims is not limited to the least square method.

According to an embodiment, the least square method is expressed as the following mathematical equation 1: $m = \frac{{\sum }_{i = I - a}^{I + b} \left({x}_{i}-\overline{x}\right) \left({y}_{i}-\overline{y}\right)}{{\sum }_{i = I - a}^{I + b} {\left({x}_{i}-\overline{x}\right)}^{2}}$ Equation 1 wherein $\overline{x} = \frac{{\sum }_{i = I - a}^{I + b} {x}_{i}}{n} \overline{y} = \frac{{\sum }_{i = I - a}^{I + b} {y}_{i}}{n}$

I is a cycle of a data point whose slope is to be calculated, m is a slope of a data point at I^{th} cycle, xᵢ is a cycle of i^{th} cycle, yᵢ is a signal value measured at i^{th} cycle,

The "n" or "a+b+1" is the number of data points used for calculating a slope at I^{th} cycle, called as LSMR (Linear Squares Method Range). The "a" is a value for calculating a minimum cycle among a set of data points used for calculating a slope at I^{th} cycle. The "b" is a value for calculating a maximum cycle.

The "a" and "b" independently represent an integer of 0-10, particularly 1-5, more particularly 1-3.

Although it is advantageous that the values of "a" and "b" are the same, they may be different from each other depending on the subject of measurement, the measurement environments and the cycle of which the slope is to be measured.

According to an embodiment, a signal change amount at a specific cycle may be provided by a different method from those of signal change amounts at other cycles, when the method for providing signal change amounts at other cycles is not suitable for providing a signal change amount at the specific cycle.

For example, where a signal change amount is obtained by a difference method, a signal change amount at the first cycle or the last cycle may be provided in a different way from those of the other cycles.

According to an embodiment, a signal change amount at the first cycle of a signal change-amount data set may be designated as a value of zero (0). In this case, a signal value in a baseline region of a reconstructed data set provided by obtaining cumulated values at each cycle using the signal change amounts of the signal change-amount data set may become a value of zero (0).

Alternatively, a signal change amount at the first cycle of a signal change-amount data set may be designated as a signal value of the first cycle of a raw data set or a predetermined signal value. In this case, a signal value in a baseline region of a reconstructed data set provided by obtaining cumulated values at each cycle using the signal change amounts of the signal change-amount data set may become the same value as the signal value of the first cycle of a raw data set or the predetermined signal value.

Before the step (c) in which a reconstructed data set is obtained by obtaining cumulated values at each cycle using a signal change amounts of a signal change-amount data set, at least one signal change amount of the signal change-amount data set may be modified. According to an embodiment, the method may further comprise the step of modifying at least one signal change-amount of the signal change-amount data set. The modification of at least one signal change amount may be a correction of an abnormal signal or a baselining of the signal change-amount data set.

The modification of a signal change-amount data set affects the reconstructed data set which is provided by obtaining cumulated values at each cycle using signal change amounts of the signal change-amount data set.

### <Baselining of the signal change-amount data set>

The baseline region of a data set refers to a region where signals are little or no affected by the presence or occurrence of a target material or target phenomenon but reflect signals generated mostly by a signal measuring device *per se* or a signal-generating reaction *per se* not related to a target analyte. Generally, there is little change in signal values in a baseline region. However, there is a data set having an error causing signal values to be varied with the cycles regardless of the presence or absence of a target analyte in the baseline region. In this case, the error in the baseline region needs to be corrected.

The conventional approaches for correcting such error in the baseline region include the following processes:
The baseline region is determined after plotting a data set and then the degree of tilt of the baseline is determined. After that, a corrected data set is obtained by rotating the plotted curve appropriately. Otherwise the corrected data set is obtained by obtaining a linear function equation fitted to the baseline region and then subtracting a corresponding output value of the function at each cycle.

These conventional approaches have some drawback such that they have to utilize very complicated processes of calculating the slope of the baseline after determining the baseline region or obtaining of a linear function fitted to the baseline.

The present method solves the baseline error problem of the data set by modifying a signal change-amount data set and obtaining cumulated values at each cycle using the signal change amounts of the modified signal change-amount data set.

The term used herein "baselining of a data set" refers to a process of modifying a data set by subtracting a value representing a baseline from a signal value at each cycles of the data set, whereby a baseline subtracted data set may be obtained.

Through the baselining of the signal change-amount data set, a value in the baseline region of the signal change-amount data set is adjusted to zero (0). The reconstructed data set may be obtained by acquiring a cumulated value at each cycle using the signal change amounts of the modified signal change-amount data set in the step (c). In this way, it is possible to obtain a modified signal change-amount data set in which the baseline error is corrected.

According to an embodiment, the signal change-amount data set may be a baseline subtracted signal change-amount data set.

The subtraction of a baseline may be an amendment of a data set according to a value in the baseline region of the data set.

Particularly, the baseline subtracted signal change-amount data set may be a signal change-amount data set in which the signal change values in the baseline region are adjusted to zero(0).

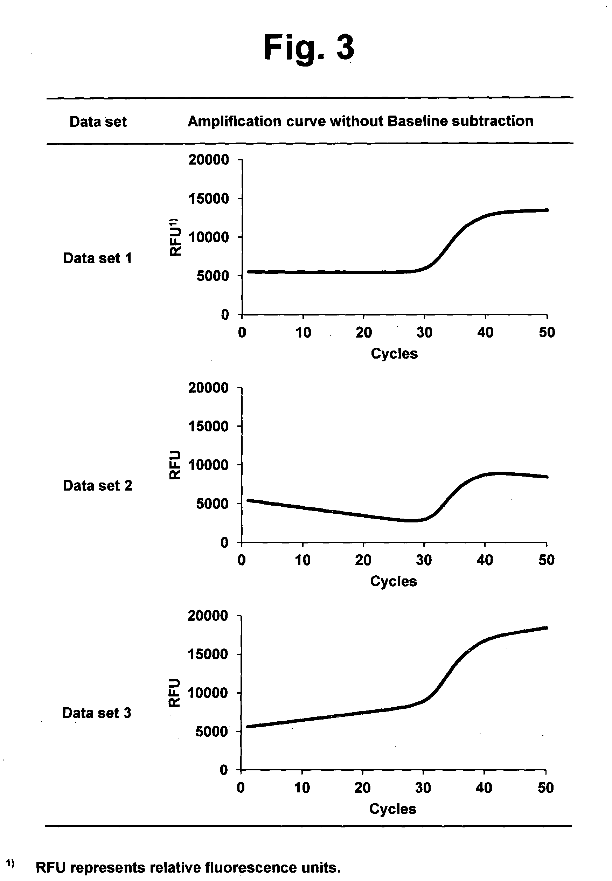

The baseline subtracted signal change-amount data set may be provided by amending at least one signal change amount of the signal change-amount data set, particularly, by subtracting a signal value of a baseline region of the signal change-amount data set from each signal change amount of the signal change-amount data set. Therefore, according to an embodiment, the present method may comprise the step of baselining a signal change-amount data set provided in the step (b) before the step (c). The baselining step is an optional step for solving a problem that a baseline of the data set is inclined. The baselining step may be usefully utilized when a baseline of the raw data set is inclined as in the data set 2 rather than in the data set 1 in Figure 3.

The method of baselining of the signal change-amount data set is not limited to any specific method and may be selected from the methods known to those skilled in the art.

According to a particular embodiment of the present invention, a specific value may be subtracted from signal change amount in each cycle of the signal change-amount data set such that the signal change amount of the data set in the baseline region has substantially a value of zero(0).

Specifically, after determining an initial specific cycle or region of cycles in the signal change-amount data set, where signals are little or no affected by the presence or occurrence of a target, the average signal change amount in the determined cycles is calculated, and then a baseline subtracted signal change-amount data set is obtained by subtracting the calculated average signal change amount from the signal change amounts at each cycle, respectively.

### <Correction of abnormal signal in the signal change-amount data set>

The correction of an abnormal signal in the signal change-amount data set is one embodiment of the modification step of the present invention. The correction of an abnormal signal in the signal change-amount data set of the present invention may be carried out by detecting a cycle having the signal value classified as an abnormal signal from a signal change-amount data set and then correcting the abnormal signal corresponding to the detected cycle thereby correcting an abnormal signal value of the raw data set.

The correction of an abnormal signal in a data set using a signal change-amount data set is described in detail in Section II.

### Step (c): Providing a reconstructed data set by obtaining cumulated values (S130)

A reconstructed data set may be provided by obtaining a cumulated value at each cycle using the signal change amounts of the signal change-amount data set.

The reconstructed data set comprises a plurality of data points comprising cycles and cumulated values at each cycle. The cumulated value is an altered value in comparison with the signal value of the original raw data set.

The reconstructed data set provided by obtaining a cumulated value at each cycle using the signal change amounts of the signal change-amount data set comprises a plurality of data points comprising an altered signal value.

The signal change-amount data set is obtained by obtaining a signal change amount using a signal value of the raw data set through steps (a) and (b), and then the signal change-amount data set is converted into the reconstructed data set by obtaining a cumulated value using the signal change amount of the signal change-amount data set through the step (c). Through the steps (a)-(c), the raw data set is converted into a reconstructed data set which is much more suitable for the detection of a target analyte.

According to an embodiment, the cumulated value at each cycle may be selected from the group consisting of a cumulated value of derivative values at each cycle; a cumulated value of differences at each cycle; a cumulated value of ratios at each cycle; and a cumulated value of slope values at each cycle.

According to an embodiment, the cumulated value at each cycle may be obtained by using a cumulation-starting cycle and a cumulation-starting value.

According to an embodiment, the cumulated value at each cycle may be calculated by one of the following calculations depending on the number of said each cycle (Xᵢ) relative to the number of a cumulation-starting cycle (CSC):
wherein the cumulation-starting cycle (CSC) is a cycle selected from the cycles of the signal change-amount data set;
(Cal-1) wherein when Xᵢ is larger than the number of CSC, the cumulated value at said each cycle is calculated by cumulating (i) a cumulation-starting value and (ii) signal change amount(s) from a cycle immediately following the cumulation-starting cycle to said each cycle; wherein the cumulation-starting value is a cumulated value at the cumulation-starting cycle;
(Cal-2) wherein when Xᵢ is smaller than the number of CSC, the cumulated value at said each cycle is calculated by cumulating (i) the cumulation-starting value and (ii) a value(s) derived from signal change amount(s) from a cycle immediately following said each cycle to the cumulation-starting cycle; and
(Cal-3) wherein when Xᵢ is equal to the number of CSC, the cumulation-starting value is designated as the cumulated value at said each cycle.

The term used herein "a value derived from a signal change amount" refers to a value obtained by modifying the signal change amount. The modification may be mathematical modification. According to an embodiment, the value derived from a signal change amount may be a mathematically modified signal change amount. For example, a value derived from signal change amount may include an additive inverse, a multiplicative inverse or a reciprocal for a signal change amount but not limited to.

The cumulated value at each cycle may be obtained by different ways depending on the number of said each cycle (Xᵢ) relative to the number of a cumulation-starting cycle (CSC).

The cumulated value may be a cumulative sum or a cumulative product.

Where the cumulated value is a cumulative sum and the number of a cycle (X cycle) at which the cumulated value is to be calculated is larger than the number of CSC, the cumulated value at the X cycle is obtained by cumulating (i) a cumulation-starting value and (ii) signal change amount(s) from a cycle immediately following the cumulation-starting cycle to the X cycle.

Where the cumulated value is a cumulative sum and the number of the X cycle is smaller than the number of CSC, the cumulated value at the X cycle is obtained by cumulating (i) a cumulation-starting value and (ii) additive inverse(s) of signal change amount(s) from a cycle immediately following the X cycle to the cumulation-starting cycle.

Where the cumulated value is a cumulative sum, said "cumulating" may be "adding".

In mathematics, the additive inverse of a number x, denoted by -x, is the number that, when added to x, yields zero.

Where the cumulated value is a cumulative product and the number of a cycle (X cycle) at which the cumulated value is to be calculated is larger than the number of CSC, the cumulated value at the X cycle is obtained by cumulating (i) a cumulation-starting value and (ii) signal change amount(s) from a cycle immediately following the cumulation-starting cycle to the X cycle.

Where the cumulated value is a cumulative product and the number of the X cycle is smaller than the number of CSC, the cumulated value at the X cycle is obtained by cumulating (i) a cumulation-starting value and (ii) the reciprocal(s) for signal change amount(s) from a cycle immediately following the X cycle to the cumulation-starting cycle.

Where the cumulated value is a cumulative product, said "cumulating" may be "multiplying".

In mathematics, a multiplicative inverse or reciprocal for a number x, denoted by 1/x, is a number which when multiplied by x yields the multiplicative identity, 1.

The term used herein "cumulation-starting cycle" refers to a cycle at which the cumulation for obtaining a cumulated value at each cycle is initiated. The cumulation-starting cycle may be arbitrarily determined. Any cycle in the data set including the first cycle and the last cycle of the data set may be designated as a cumulation-starting cycle.

According to an embodiment, the cumulation-starting cycle may be the first cycle of a data set. In this case, the cumulated value at each cycle may be calculated by cumulating the signal change amounts from the first cycle up to said each cycle.

Where a cycle in the baseline region of the data set is designated as a cumulation-starting cycle *(e.g.,* the first cycle is designated as a cumulation-starting cycle), a cumulation-starting value may be a signal value in a baseline region of the reconstructed data set. In this case, a baseline-subtracted reconstructed data set may be easily obtained by assigning a value of zero to the cumulation-starting value.

The term used herein "cumulation-starting value" refers to a signal value at cumulation-starting cycle of the reconstructed data set. The cumulation-starting value may be arbitrarily determined.

According to an embodiment, the cumulation-starting value may be a signal value at a cumulation-starting cycle of raw data set. A reconstructed data set similar to the raw data set in view of a signal value at each cycle may be obtained by assigning a signal value at a cumulation-starting cycle of raw data set to the cumulation-starting value.

According to another embodiment, the cumulation-starting value may be a value of zero ("0") or one ("1"). When the cumulation-starting cycle is determined within a baseline region, a baseline subtraction of the reconstructed data set is easily performed by assigning a value of zero to the cumulation-starting value.

Where the cumulated values of a reconstructed data set are obtained by finding an integral of a function fitting a signal change-amount data set, the cumulated values may be obtained by (i) dividing the cycles of the signal change-amount data set into two groups based on the cumulation-starting cycle *(i.e.,* a group of cycles before or after the cumulation-starting cycle) and then (ii) finding an integral of a separate fitting function for each group. A constant of integration may be determined such that an integral value of a fitting function at the cumulation-starting cycle of a signal change-amount data set would be a cumulation-starting value.

In addition, the cumulation-starting value may be a signal value at the cumulation-starting cycle of a signal change-amount data set.

According to an embodiment, the cumulated value at each cycle is calculated by cumulating the signal change amounts from the first cycle up to said each cycle.

Where the cumulation-starting cycle is the first cycle of a signal change-amount data set and the cumulation-starting value is a signal value at the cumulation-starting cycle, the cumulated value at each cycle is calculated simply by cumulating the signal change amounts from the first cycle up to said each cycle.

A phrase "cumulating the signal change amounts from the first cycle up to said each cycle" may include "multiplying the signal change amounts from the first cycle up to the each cycle" and "adding the signal change amounts from the first cycle up to the each cycle".

Where a signal change amount is a derivative value of signal values at each cycle, a difference in signal values with regard to a previous cycle at each cycle or a slope value obtained by a linear regression analysis at each cycle, and the cumulation-starting cycle is the first cycle of a signal change-amount data set and the cumulation-starting value is a signal value at the cumulation-starting cycle, a cumulated value may be a cumulative sum calculated by adding the signal change amounts from the first cycle up to the each cycle. For example, a cumulative sum at 10^{th} cycle (*i*.*e*., cycle number 10) is calculated by adding all of the signal change amounts at cycle numbers from 1 to 10. Alternatively, a cumulative sum at each cycle may be obtained by finding an integral of a function fitting a signal change-amount data set. A constant of integration may be designated as zero(0) or an arbitrary constant.

When the constant of integration is designated as zero(0), a baseline of the reconstructed data set may be adjusted to a value of zero(0) or substantially zero. When the constant of integration is designated as a signal value of a baseline region of a raw data set, a reconstructed data set of which a baseline is of the same value as a baseline of a raw data set may be obtained.

The cumulative product may be calculated by multiplying the signal change amounts from the first cycle up to the each cycle.

Where a signal change amount is a ratio of signal values with regard to a previous cycle at each cycle, and the cumulation-starting cycle is the first cycle of a signal change-amount data set and the cumulation-starting value is a signal value at the cumulation-starting cycle, a cumulated value may be a cumulative product calculated by multiplying the signal change amounts from the first cycle up to the each cycle.

According to an embodiment, a signal change amount at a specific cycle may be provided by a calculation method different from one used for calculating signal change amounts at other cycles. Such embodiment may be useful when the calculation method used for signal change amounts at other cycles is not suitable for providing a signal change amount at the specific cycle.

Accordingly, a cumulated value of the reconstructed data set may be varied according to methods for calculating the signal change amounts when the cumulation-starting value is designated as a signal value at the cumulation-starting cycle of a signal change-amount data set.

For example, where the cumulation-starting cycle is the first cycle of a data set, the cumulation-starting value is a signal value at the cumulation-starting cycle of a signal change-amount data set and a signal change amount at the first cycle of a signal change-amount data set is designated as a value of zero (0), a cumulated value at the first cycle of a reconstructed data set also has a value of zero (0) and cumulated values at the following cycles are determined based on the cumulated value at the first cycle.

For another example, where the cumulation-starting cycle is the first cycle of a data set, the cumulation-starting value is a signal value at the cumulation-starting cycle of a signal change-amount data set and a signal change amount at the first cycle of a signal change-amount data set is designated as the same value as a signal value at the first cycle of a raw data set, a cumulated value at the first cycle of a reconstructed data set also has the same value as a signal value at the first cycle of a raw data set and cumulated values at the following cycles are determined based on the cumulated value at the first cycle.

According to the method of the present invention, regardless of what the initial signal value of the baseline region of the raw data set is, a reconstructed data set in which a signal value of a baseline region has a specific value (*e*.*g*., a value of zero or the same value as a signal value at the first cycle of a raw data set) can be obtained. Therefore, a plurality of data sets may be corrected with an identical criterion by the present method and thus a reliable analysis result can be obtained.

According to an embodiment, the method further comprises the step of repeating the steps (b)-(c) for smoothing the data set in which the reconstructed data set provided by the step (c) is used as the data set for the step (b).

The term used herein "smoothing of the data set" refers to a processing or refining of the data set by a predetermined algorithm to remove or minimize a noise of the data set. The visual representation of the data set is improved by the smoothing of the data set. The smoothing of the data set is described in detail in Section IV.

### Step (d): Detecting the target analyte by using the reconstructed data set (S140)

The target analyte in a sample is detected by analyzing the reconstructed data set

The detection of the target analyte in a sample means a qualitative or quantitative detection of a target analyte in a sample using a data set obtained by a signal-generating process for a target analyte. The qualitative or quantitative detection comprises a detection of presence or absence of a target analyte in a sample, a detection of an amount of a target analyte in a sample, a detection of changes in amount or state of a target analyte by a biological or chemical reaction.

The terms "analysis of a target analyte", "detection of a target analyte" or "qualitative or quantitative analysis of a target analyte" or "qualitative or quantitative detection of a target analyte" refer to an acquisition of information on presence or absence of a target analyte in a sample; an amount of a target analyte in a sample; or changes in amount or state of a target analyte by a biological or chemical reaction and These terms are used interchangeably.

According to an embodiment, the detection of the target analyte of the step (d) may be a qualitative or quantitative detection of the target analyte in the sample

In another aspect of this invention, there is provided a method for detecting a target analyte in a sample comprising:
(a) providing a data set for a target analyte;
(b) normalizing the data set;
(c) providing a signal change-amount data set by obtaining a signal change amount at each cycle using the signal values of the data set;
(d) baselining the signal change-amount data set;
(e) amending the signal change-amount data set;
(f) providing a reconstructed data set by obtaining a cumulated value at each cycle using the signal change amounts of the amended signal change-amount data set; and
(g) detecting the target analyte in the sample by using the reconstructed data set.

Since all the steps in the target analyte detection method including the steps (a) to (g) are described above, the common descriptions between them are omitted in order to avoid undue redundancy leading to the complexity of this specification.

According to the target analyte detection method including the steps (a) to (g) comprising the steps of normalizing a data set, amending a signal change-amount data set and providing a reconstructed data set, most of problems associated with detection of target analyte by signal-generating process such as signal variation between instruments, abnormal signal, noise, abnormal baseline can be solved. In the above steps, steps of (b), (d), and (e) may be appropriately selected and used in combination.

### II. Method for Detecting a Target Analyte in a Sample Comprising Amendment and Transformation of Signal change-amount data set

A processed data set suitable for detecting a target analyte in a sample may be obtained by modification and transformation of a signal change-amount data set.

Generally, the signal change-amount data set is used for providing information needed for direct correction of a raw data set. For instance, the signal change-amount data set is used to provide information for identifying an end point of the baseline region, or information for determining whether a signal at each cycle is an abnormal signal, but the correction of the baseline region or the correction of the abnormal signal is carried out by direct modification of the original data set, not by modification of the signal change-amount data set.

However, according to the present invention, the correction of the baseline region or the correction of the abnormal signal of the original data set is performed by the modification of a signal change-amount data set, and the effect of the modification of a signal change-amount data set can be transferred to a finally processed data set for the target analyte detection through a transformation process of the modified signal change-amount data set.

Since the validation of a signal value at each cycle and the modifications for securing the validity of a data set can be made in the same data set by the present method, it is possible to correct the data set more accurately and efficiently and to detect the target analyte with improved reliability using the modified data set.

In one aspect of this invention, there is provided a method for detecting a target analyte in a sample comprising:
(a) providing a data set for the target analyte; wherein the data set comprises a plurality of data points comprising cycles and signal values of the signal-generating process;
(b) providing a signal change-amount data set by obtaining a signal change amount at each cycle using the signal values of the data set; wherein the signal change-amount data set comprises a plurality of data points comprising cycles and signal change-amounts at the cycles;
(c) providing an amended signal change-amount data set by amending directly the signal change-amount data set;
(d) providing a transformed data set by transforming the amended signal change-amount data set; and
(e) detecting the target analyte in the sample by using the transformed data set.

Through the steps (a) and (b), a data set for the target analyte is obtained and a signal change-amount data set is obtained by calculating a signal change amount at each cycle number therefrom. The steps (a) and (b) are as described in section I.

### Step (c): Providing an amended signal change-amount data set by amending the signal change-amount data set

In the step (c), an amended signal change-amount data set is provided by amendment of a signal change-amount data set.

As an embodiment of the amendment of a signal change-amount data set, the amendment may comprises the steps of detecting a cycle having an abnormal signal value using a signal change-amount data set and then correcting a signal change amount corresponding to the detected cycle, thereby correcting the abnormal signal value.

According to an embodiment, the amendment of a signal change-amount data set to correct an abnormal signal is performed as following method:

### <Method for correcting an abnormal signal using a signal change-amount data set>

### - Detection of an abnormal signal

Abnormal signals in a data set are accompanied by abnormal signal changes. According to a method of present invention, an abnormal signal is corrected by detecting an abnormal signal change value and correcting it by using a signal change-amount data set.

As a first step of detecting a peak indicating an abnormal signal change (abnormal peak), peaks in the signal change-amount data set are recognized. The peak refers to a point or a local section containing a local maximum or minimum value (*i*.*e*., turning point) wherein the point or the local section is a data point or a group of two or more consecutive data points.

Specifically, the data point or the group of two or more consecutive data points may have signal value that deviates from a predetermined criterion. For example, the criterion may be a threshold of specific value. In this case, two or more consecutive data points that have a value more than the threshold may be determined as a peak. For another example, the criterion may be a predetermined specific ratio with respect to a maximum value or a minimum value. In this case, two or more consecutive data points that have a ratio value greater than the predetermined ratio may be determined as a peak.

In one embodiment of the present invention, a peak is identified by using a threshold value. Specifically, a group of two or more consecutive data points of a signal change-amount data set that contain a local maximum or minimum value and have a signal change value more than the threshold may be recognized as one peak.

As a second step of detecting a peak indicating an abnormal change (abnormal peak), it is determined whether the peak is a normal peak that represents a true signal change value or an abnormal peak.

In an embodiment, a "Half Peak Width" method is used to determine whether a peak of a signal change-amount data set is a normal peak or an abnormal peak. According to the Half Peak Width method, abnormality of the peak is determined by using a half width of a peak.

A max cycle number is a cycle number of a data point of a signal change-amount data set having a maximum signal change-amount in a peak and a start cycle number is a cycle number of the first data point in the peak exceeding a threshold value. The half width of a peak is a difference (Δcycle) between the max cycle number and the start cycle number.

An abnormal signal generated by a noise or other abnormal environments generally exhibits a pattern of signal change such that a signal value is increased or decreased more sharply as compared with a normal signal generated by a target analyte. By analyzing such pattern of signal changes, it is possible to distinguish whether the peak is a normal peak or an abnormal one. Usually, the half peak width of an abnormal peak is smaller than that of a normal peak. According to an embodiment, a peak whose half width of a peak is less than a predetermined threshold is determined as an abnormal peak.

### - Correction of the abnormal signal

The abnormal signal detected may be corrected through the amendment of the signal change-amount data set.

According to an embodiment of the present invention, an abnormal signal may not be directly corrected but may be corrected by amending the signal change-amount of the signal change-amount data set obtained from the raw data set to eliminate errors that may occur in the target analyte detection.

The signal change amounts in the abnormal peak may be amended to have the same value to each other or may be amended to increase or decrease at a constant rate as the cycle number increases or decreases.

When the signal change amounts in the abnormal peak are amended to have the same value to each other, the signal values of the data points corresponding to the abnormal peak are corrected to increase as the same rate such that an abnormal signal is eliminated.

When the signal change-amount of cycles before and after the abnormal peak have the same or similar values, the signal change-amounts within the abnormal peak can be amended to the same or similar values with the signal change-amount before and after the abnormal peak. Specifically, the average value of the signal change-amounts of the one or more cycles before and after the abnormal peak may be designated as signal change-amounts within the abnormal peak. Particularly, such an amendment may be applied when the data points of an abnormal peak correspond to an amplification region in a data set.

According to an embodiment, the signal change-amounts within the abnormal peak may be amended to have a value of zero(0). By such an amendment, the signal values of data points corresponding to the abnormal peak are not increased but have same values with a signal value of immediately previous data point. Particularly, such an amendment may be applied when data points of abnormal peak correspond to a background region in a data set. At least one signal change-amount within the abnormal peak may be amended to have a value of zero(0). According to an embodiment, only signal change-amounts that exceed a threshold may be amended to have a value of zero(0).

### <Noise correction of a background region>

According to an embodiment, signal change-amounts in a background region may be amended to correct a noise signal of the background region. Since this step is not for correcting the abnormal peak but is an optional step for correcting the noise of the background signal, it may be performed after the abnormal peak detection and the amendment of the signal change-amount of the abnormal peak described above. Otherwise, the step may be performed independently.

The term used herein "noise" refers to an unwanted and non-analyte related signal that occurs independently of the presence or absence of a target analyte.

Since remaining peaks in a signal change-amount data set after an amendment of the abnormal peaks may be normal peaks, the signal change-amounts of cycles before the first cycle of the first normal peak may be amended to have a value of zero (0).

The determination of the first cycle of the normal peak can be determined in various ways.

According to an embodiment, a gap may be applied to determine the first cycle of the normal peak. A gap is a number of interval cycles between a first cycle of the normal peak and a start cycle which is a cycle of the first data point exceeding a threshold value in the normal peak.

When the normal peak is recognized by the threshold value, the first cycle of the normal peak may be determined by subtracting the gap from the cycle number corresponding to the first data point exceeding a threshold value in the normal peak.

The gap may be appropriately predetermined according to the general signal pattern of the signal generation reaction and the level of the threshold value applied.

In an embodiment of the present invention, the first cycle of the normal peak is determined using 5 as a gap for recognizing the cycle number immediately before the normal peak is generated.

### Step (d): Providing a transformed data set by transforming the amended change-amount data set

In the step (d), a transformed data set is provided by transforming the amended change-amount data set. The transformed data set is used for detecting a target analyte in a sample.

The transformation of the amended change-amount data set may be performed in order to provide a data set having more suitable structure for detecting a target analyte in a sample.

According to an embodiment, the amended signal change-amount data set may be transformed to a reconstructed data set comprising data points having cycles and corresponding cumulated value and then the target analyte may be detected using the reconstructed data set. The cumulated value corresponding to each cycle in the reconstructed data set is a value of a signal which uses the same unit (*e*.*g*., RUF) with the raw data set. Therefore, the conventional criteria used for detecting a target analyte from a raw data set can be applied to a reconstructed data set for detecting a target analyte.

According to an embodiment, the transformed data set may be a ***N***^{th} order signal change-amount data set comprising ***N***^{th} order signal change-amounts representing changes of signal change-amounts of the amended signal change-amount data set; wherein ***N*** represents an integer of more than 2. The higher order signal change-amount data set can specify the first cycle at which a change (*i*.*g*., an increase or a decrease) is started more accurately as compared with the change value data set.

### Step (e): Detecting the target analyte in the sample by using the transformed data set

In the step (e), the target analyte in the sample is detected by using the transformed data set.

The detection of the target analyte in a sample means a qualitative or quantitative detection of a target analyte in a sample using a data set obtained by a signal-generating process for a target analyte. The qualitative or quantitative detection comprises a detection of presence or absence of a target analyte in a sample, a detection of an amount of a target analyte in a sample, a detection of changes in amount or state of a target analyte by a biological or chemical reaction.

The terms used herein "analysis of a target analyte", "detection of a target analyte" or "qualitative or quantitative analysis of a target analyte" or "qualitative or quantitative detection of a target analyte" refer to an acquisition of information on the presence or absence of a target analyte in a sample; an amount of a target analyte in a sample; or changes in amount or state of a target analyte by a biological or chemical reaction and these terms are used interchangeably.

According to an embodiment, the detection of the target analyte of the step (e) may be a qualitative or quantitative detection of the target analyte in the sample.

The detection method of the present invention may be appropriately selected depending on the type of the obtained transformed data set.

For example, when a transformed data set is a reconstructed data set provided by obtaining a cumulated value at each cycle using the signal change amounts of the signal change-amount data set, a reference cycle number for detecting the target analyte(*i*.*e*., Cₜ : a threshold cycle) may be determined by predetermined threshold value.

For another example, when the transformed data set is a 2^{nd} order signal change-amount data set provided by obtaining a changed value of the signal change-amount at each cycle using the signal change amounts of the signal change-amount data set, a cycle number corresponding to a maximum value of the first peak of 2^{nd} order signal change-amount data set may be designated as a threshold cycle (Ct).

### III. Method for Reconstructing a Data Set

In one aspect of this invention, there is provided a method for reconstructing a data set comprising:
(a) providing a signal change-amount data set by obtaining a signal change amount at each cycle using signal values of a data set; wherein the data set comprises a plurality of data points comprising cycles and signal values of a signal-generating process; wherein the signal change-amount data set comprises a plurality of data points comprising cycles and signal change-amounts at the cycles; and
(b) providing a reconstructed data set by obtaining a cumulated value at each cycle using the signal change amounts of the signal change-amount data set; wherein the reconstructed data set comprises a plurality of data points comprising cycles and cumulated values at the cycle.

Since all of the steps described in the method of Section III overlap with those of Section I, the common descriptions between them are omitted in order to avoid undue redundancy leading to the complexity of this specification.

According to an embodiment, the signal change amount at each cycle is selected from the group consisting of a derivative value of the signal values at each cycle; a difference in the signal values at each cycle; a ratio of the signal values at each cycle; and a slope value obtained by performing a linear regression analysis at each cycle.

According to an embodiment, the cumulated value at each cycle is selected from the group consisting of a cumulated value of derivative values at each cycle; a cumulated value of differences at each cycle; a cumulated value of ratios at each cycle; and a cumulated value of slope values at each cycle.

According to an embodiment, the present method may comprise a baselining step of the signal change-amount data set. The baselining of the signal change-amount data set is as described in section I.

The data set can be calibrated through a method for reconstructing a data set that comprise the steps of converting a data set to a signal change-amount data set and then reconverting the signal change-amount data set into a reconstructed data set. Through such a reconstruction, a data set is processed to be suitable for detection of the target analyte.

### IV. Method for Smoothing a Data Set

In one aspect of this invention, there is provided a method for smoothing a data set comprising:
(a) providing a data set for a target analyte; wherein the data set is obtained from a signal-generating process for the target analyte using a signal-generating means; wherein the data set comprises a plurality of data points comprising cycles and signal values of the signal-generating process;
(b) providing a signal change-amount data set by obtaining a signal change amount at each cycle using the signal values of the data set; wherein the signal change-amount data set comprises a plurality of data points comprising the cycles and signal change-amounts at the cycles; and
(c) providing a reconstructed data set by obtaining a cumulated value at each cycle using the signal change amounts of the signal change-amount data set; wherein the reconstructed data set comprises a plurality of data points comprising cycles and cumulated values at the cycles.

According to an embodiment, the method for smoothing a data set may further comprise the step of repeating the steps (b)-(c) for smoothing the data set in which the reconstructed data set provided by the step (c) is used as the data set for the step (b).

The term used herein "smoothing of the data set" refers to a processing or refining of the data set by a predetermined algorithm to remove or minimize a noise or variation of the data set. The visual representation of the data set may be improved by the smoothing of the data set.

In data analysis, the smoothing of a data set is a creation of an approximate data set that holds the major patterns of the data set. The smoothing of the data set removes a noise or other fine structures or sudden phenomena. Through the smoothing process, a signal at a data point is modified so that the characteristics of individual data points are reduced and the signal difference from the adjacent points is reduced. This makes it easy to recognize information about the macroscopic change of signals in a data set.

Since the steps of (a)-(c) described in the method of Section IV overlap with those of Section I, the common descriptions between them are omitted in order to avoid undue redundancy leading to the complexity of this specification.

According to an embodiment, the signal change amount may be a slope value obtained by performing a linear regression analysis at each cycle.

The linear regression analysis is described above in Section I.

Where a signal change-amount is obtained by the linear regression analysis, the difference between the signal values of the cycles adjacent to the cycle at which a signal change-amount to be calculated since the signal change-amount at the cycle is obtained by using a signal value corresponding to the several cycles before and after the cycle at which a signal change-amount to be calculated.

A reconstructed data set obtained from the signal change-amount data set by using this method has more alleviated deviation of the signal value between the adjacent cycles than the previous data set (*e*.*g*., a data set before being processed for obtaining the signal change-amount data set) so that the reconstructed data set exhibits a more smooth curve.

As a method of linear regression analysis, a least square method may be used but not limited to.

The below descriptions illustrate a least square method as a representative of a linear regression analysis but the scope of the present invention as set forth in the appended claims is not limited to the least square method.

According to an embodiment, the least square method is expressed as the following mathematical equation 2: $m = \frac{{\sum }_{i = I - a}^{I + b} \left({x}_{i}-\overline{x}\right) \left({y}_{i}-\overline{y}\right)}{{\sum }_{i = I - a}^{I + b} {\left({x}_{i}-\overline{x}\right)}^{2}}$ Equation 2 wherein $\overline{x} = \frac{{\sum }_{i = I - a}^{I + b} {x}_{i}}{n} \overline{y} = \frac{{\sum }_{i = I - a}^{I + b} {y}_{i}}{n}$

I is a cycle of a data point whose slope is to be calculated, m is a slope of a data point at I^{th} cycle, xᵢ is a cycle of i^{th} cycle, yᵢ is a signal value measured at i^{th} cycle,
The "n" or "a+b+1" is the number of data points used for calculating a slope at I^{th} cycle, called as LSMR (Linear Squares Method Range). The "a" is a value for calculating a minimum cycle among a set of data points used for calculating a slope at I^{th} cycle. The "b" is a value for calculating a maximum cycle.

The "a" and "b" independently represent an integer of 0-10, particularly 1-5, more particularly 1-3.

Although it is advantageous that the values of "a" and "b" are the same, they may be different from each other depending on the subject of measurement, the measurement environments and the cycle of which the slope is to be measured.

For example, the values of "a" and "b" (*i*.*e*., (a, b)) may be selected from a group of (1, 1), (2, 2), (3, 3) and (4, 4). The first value of the ordered pair represents the value of "a" and the second value represents the value of "b".

Where a change (increase or decrease) of the signal value is increased depending on the presence or amount of a target analyte, to prevent the change starting cycle from shifting by the smoothing process, a value of "a" may be set to be larger than the value of "b". For example, the values of "a" and "b" may be selected from a group of (2, 1), (3, 1), (4, 1), (5, 1), (3, 2), (4, 2), (5, 2), (4, 3), (5, 3) and (5, 4).

On the contrary, where a change (increase or decrease) of the signal value is decreased depending on the presence or amount of a target analyte, to prevent the change starting cycle from shifting by the smoothing process, a value of "b" may be set to be larger than the value of "a". For example, the values of "a" and "b" may be selected from a group of (1, 2), (1, 3), (1, 4), (1, 5), (2, 3), (2, 4), (2, 5), (3, 4), (3, 5) and (4, 5).

According to an embodiment, the number of data points used to obtain a slope value by performing a linear regression analysis at each cycle may be 3 to 10.

According to an embodiment, the method may further comprise the step of repeating the steps (b)-(c) for smoothing the data set in which the reconstructed data set provided by the step (c) is used as the data set for the step (b).

The repeating of steps (b)-(c) may be performed by providing a signal change-amount data set from the reconstructed data set previously obtained and providing a reconstructed data set again using the signal change-amount data set. The repetition of providing a change value data set and conversion thereof is called an Iteration Method for Data Conversion (IMDC).

The number of repetitions of the steps (b) to (c) for the smoothing of the data set is not particularly limited. For example, the number of repetition may be 1, 2, 3, 4, 5, 6, 7, 8, 9 or 10 times. In an embodiment, the number of repetition may be 1 to 10 times, 2 to 5 times, 2 to 4 times or 2 to 3 times. As the number of repetitions increases, the degree of smoothing of the data set increases. However, in the case of excessive repetition, it may be difficult to distinguish between a signal pattern indicating the presence of target analyte and a signal pattern indicating absence of target analyte.

### V. Storage Medium, Device and Computer Program

In another aspect of this invention, there is provided a computer readable storage medium containing instructions to configure a processor to perform a method for detecting a target analyte in a sample, the method comprising:
(a) receiving a data set for the target analyte;
   wherein the data set is obtained from a signal-generating process for the target analyte using a signal-generating means; wherein the data set comprises a plurality of data points comprising cycles and signal values of the signal-generating process;
(b) providing a signal change-amount data set by obtaining a signal change amount at each cycle using the signal values of the data set; wherein the signal change-amount data set comprises a plurality of data points comprising the cycles and signal change-amounts at the cycles;
(c) providing a reconstructed data set by obtaining a cumulated value at each cycle using the signal change amounts of the signal change-amount data set; wherein the reconstructed data set comprises a plurality of data points comprising cycles and cumulated values at the cycle; and
(d) determining the presence or absence of the target analyte in the sample by using the reconstructed data set.

In another aspect of this invention, there is provided a computer program to be stored on a computer readable storage medium to configure a processor to perform a method for detecting a target analyte in a sample, the method comprising:
(a) receiving a data set for the target analyte;
   wherein the data set is obtained from a signal-generating process for the target analyte using a signal-generating means; wherein the data set comprises a plurality of data points comprising cycles and signal values of the signal-generating process;
(b) providing a signal change-amount data set by obtaining a signal change amount at each cycle using the signal values of the data set; wherein the signal change-amount data set comprises a plurality of data points comprising the cycles and signal change-amounts at the cycles;
(c) providing a reconstructed data set by obtaining a cumulated value at each cycle using the signal change amounts of the signal change-amount data set; wherein the reconstructed data set comprises a plurality of data points comprising cycles and cumulated values at the cycle; and
(d) determining the presence or absence of the target analyte in the sample by using the reconstructed data set.

The program instructions are operative, when performed by the processor, to cause the processor to perform the present method described above. The program instructions for performing the method for detecting a target analyte in a sample may comprise an instruction to receive a data set for the target analyte; an instruction to provide a signal change-amount data set by obtaining a signal change amount at each cycle using the signal values of the data set; an instruction to provide a reconstructed data set by obtaining a cumulated value at each cycle using the signal change amounts of the signal change-amount data set and an instruction to determine the presence or absence of the target analyte in the sample by using the reconstructed data set.

In another aspect of this invention, there is provided a computer readable storage medium containing instructions to configure a processor to perform a method for detecting a target analyte in a sample, the method comprising:
(a) receiving a data set for the target analyte; wherein the data set comprises a plurality of data points comprising cycles and signal values of the signal-generating process;
(b) providing a signal change-amount data set by obtaining a signal change amount at each cycle using the signal values of the data set; wherein the signal change-amount data set comprises a plurality of data points comprising cycles and signal change-amounts at the cycles;
(c) providing an amended signal change-amount data set by amending directly the signal change-amount data set;
(d) providing a transformed data set by transforming the amended signal change-amount data set; and
(e) determining the presence or absence of the target analyte in the sample by using the transformed data set.

In another aspect of this invention, there is provided a computer program to be stored on a computer readable storage medium to configure a processor to perform a method for detecting a target analyte in a sample, the method comprising:
(a) receiving a data set for the target analyte; wherein the data set comprises a plurality of data points comprising cycles and signal values of the signal-generating process;
(b) providing a signal change-amount data set by obtaining a signal change amount at each cycle using the signal values of the data set; wherein the signal change-amount data set comprises a plurality of data points comprising cycles and signal change-amounts at the cycles;
(c) providing an amended signal change-amount data set by amending directly the signal change-amount data set;
(d) providing a transformed data set by transforming the amended signal change-amount data set; and
(e) determining the presence or absence of the target analyte in the sample by using the transformed data set.

The program instructions are operative, when performed by the processor, to cause the processor to perform the present method described above. The program instructions for performing the method for detecting a target analyte in a sample may comprise an instruction to receive a data set for the target analyte; an instruction to provide a signal change-amount data set by obtaining a signal change amount at each cycle using the signal values of the data set; an instruction to an amended signal change-amount data set by amending directly the signal change-amount data set; an instruction to provide a transformed data set by transforming the amended signal change-amount data set and an instruction to determine the presence or absence of the target analyte in the sample by using the transformed data set.

In another aspect of this invention, there is provided a computer readable storage medium containing instructions to configure a processor to perform a method for reconstructing a data set, the method comprising:
(a) receiving a data set for the target analyte; wherein the data set comprises a plurality of data points comprising cycles and signal values of a signal-generating process;
(b) providing a signal change-amount data set by obtaining a signal change amount at each cycle using signal values of the data set; wherein the signal change-amount data set comprises a plurality of data points comprising cycles and signal change-amounts at the cycles; and
(c) providing a reconstructed data set by obtaining a cumulated value at each cycle using the signal change amounts of the signal change-amount data set; wherein the reconstructed data set comprises a plurality of data points comprising cycles and cumulated values at the cycle.

In another aspect of this invention, there is provided a computer program to be stored on a computer readable storage medium to configure a processor to perform a method for reconstructing a data set, the method comprising:
(a) receiving a data set for the target analyte; wherein the data set comprises a plurality of data points comprising cycles and signal values of a signal-generating process;
(b) providing a signal change-amount data set by obtaining a signal change amount at each cycle using signal values of the data set; wherein the signal change-amount data set comprises a plurality of data points comprising cycles and signal change-amounts at the cycles; and
(c) providing a reconstructed data set by obtaining a cumulated value at each cycle using the signal change amounts of the signal change-amount data set; wherein the reconstructed data set comprises a plurality of data points comprising cycles and cumulated values at the cycle.

The program instructions are operative, when performed by the processor, to cause the processor to perform the present method described above. The program instructions for performing the method for reconstructing a data set may comprise an instruction to receive a data set for the target analyte; an instruction to provide a signal change-amount data set by obtaining a signal change amount at each cycle using the signal values of the data set; an instruction to provide a reconstructed data set by obtaining a cumulated value at each cycle using the signal change amounts of the signal change-amount data set.

In another aspect of this invention, there is provided a computer readable storage medium containing instructions to configure a processor to perform a method for smoothing a data set, the method comprising:
(a) receiving a data set for the target analyte; wherein the data set is obtained from a signal-generating process for the target analyte using a signal-generating means; wherein the data set comprises a plurality of data points comprising cycles and signal values of the signal-generating process;
(b) providing a signal change-amount data set by obtaining a signal change amount at each cycle using the signal values of the data set; wherein the signal change-amount data set comprises a plurality of data points comprising the cycles and signal change-amounts at the cycles; and
(c) providing a reconstructed data set by obtaining a cumulated value at each cycle using the signal change amounts of the signal change-amount data set; wherein the reconstructed data set comprises a plurality of data points comprising cycles and cumulated values at the cycles.

In another aspect of this invention, there is provided a computer program to be stored on a computer readable storage medium to configure a processor to perform a method for smoothing a data set, the method comprising:
(a) receiving a data set for the target analyte; wherein the data set is obtained from a signal-generating process for the target analyte using a signal-generating means; wherein the data set comprises a plurality of data points comprising cycles and signal values of the signal-generating process;
(b) providing a signal change-amount data set by obtaining a signal change amount at each cycle using the signal values of the data set; wherein the signal change-amount data set comprises a plurality of data points comprising the cycles and signal change-amounts at the cycles; and
(c) providing a reconstructed data set by obtaining a cumulated value at each cycle using the signal change amounts of the signal change-amount data set; wherein the reconstructed data set comprises a plurality of data points comprising cycles and cumulated values at the cycles.

The program instructions are operative, when performed by the processor, to cause the processor to perform the present method described above. The program instructions for performing the method for smoothing a data set may comprise an instruction to receive a data set for the target analyte; an instruction to provide a signal change-amount data set by obtaining a signal change amount at each cycle using the signal values of the data set; an instruction to providing a reconstructed data set by obtaining a cumulated value at each cycle using the signal change amounts of the signal change-amount data set.

The present method described above is implemented in a processor, such as a processor in a stand-alone computer, a network attached computer or a data acquisition device such as a real-time PCR machine.

The types of the computer readable storage medium include various storage medium such as CD-R, CD-ROM, DVD, flash memory, floppy disk, hard drive, portable HDD, USB, magnetic tape, MINIDISC, nonvolatile memory card, EEPROM, optical disk, optical storage medium, RAM, ROM, system memory and web server.

The data set may be received through several mechanisms. For example, the data set may be acquired by a processor resident in a PCR data acquiring device. The data set may be provided to the processor in a real time as the data set is being collected, or it may be stored in a memory unit or buffer and provided to the processor after the experiment has been completed. Similarly, the data set may be provided to a separate system such as a desktop computer system via a network connection (e.g., LAN, VPN, intranet and Internet) or direct connection (e.g., USB or other direct wired or wireless connection) to the acquiring device, or provided on a portable medium such as a CD, DVD, floppy disk, portable HDD or the like to a stand-alone computer system. Similarly, the data set may be provided to a server system via a network connection (e.g., LAN, VPN, intranet, Internet and wireless communication network) to a client such as a notebook or a desktop computer system.

The instructions to configure the processor to perform the present invention may be included in a logic system. The instructions may be downloaded and stored in a memory module *(e.g.,* hard drive or other memory such as a local or attached RAM or ROM), although the instructions can be provided on any software storage medium such as a portable HDD, USB, floppy disk, CD and DVD. A computer code for implementing the present invention may be implemented in a variety of coding languages such as C, C++, Java, Visual Basic, VBScript, JavaScript, Perl and XML. In addition, a variety of languages and protocols may be used in external and internal storage and transmission of data and commands according to the present invention.

In still further aspect of this invention, there is provided a device for detecting a target analyte in a sample, comprising (a) a computer processor and (b) the computer readable storage medium described above coupled to the computer processor.

In still further aspect of this invention, there is provided a device for reconstructing a data set, comprising (a) a computer processor and (b) the computer readable storage medium described above coupled to the computer processor

In still further aspect of this invention, there is provided a device for smoothing a data set, comprising (a) a computer processor and (b) the computer readable storage medium described above coupled to the computer processor

According to an embodiment, the device further comprises a reaction vessel to accommodate the sample and signal-generating means, a temperature controlling means to control temperatures of the reaction vessel and/or a detector to detect signals at amplification cycles.

According to an embodiment, the computer processor permits not only to receive values of signals at cycles but also to process a data set, provide a reconstructed data set or determine presence or absence of a target analyte in a sample.

The processor may be prepared in such a manner that a single processor can do all performances described above. Alternatively, the processor unit may be prepared in such a manner that multiple processors do multiple performances, respectively.

According to an embodiment, the processor may be embodied by installing software into conventional devices for detection of target nucleic acid molecules (e.g. real-time PCR device).

The features and advantages of this invention will be summarized as follows:
(A) The data set for detecting a target analyte is converted into a signal change-amount data set and then reconstructed by obtaining a cumulated value at each cycle using the signal change amounts of the signal change-amount data set. Therefore, a data set amendment for target analyte detection such as baselining and smoothing of a data set can be easily achieved without complicated steps such as setting a baseline region.
(B) In conventional methods for detecting a target analyte, the signal change-amount data set is used only for providing information needed for direct correction of an original data set and the correction of the data set is performed directly on the original data set based on the obtained information.

According to the present invention, necessary corrections realized by the analysis of a signal change-amount data set are directly applied to the signal change-amount data set, and alternatively the necessary corrections are applied to the data set to be used for target detection through the conversion of the signal change-amount data set.

In this way, it is possible to apply the processing for the target analyte detection, such as error correction and baselining, to the data set without directly amending the signal values of the data set. The method for detecting a target analyte including steps of providing signal change-amount data set and a reconstructed data set can effectively detect and remove abnormal signals included in the data set.

(c) An error such as a jump error generates an abnormal signal throughout the subsequent cycles as well as the cycle in which the error occurs. According to the method of present invention including a correction of an abnormal signal through the signal change-amount data set, the error such as a jump error can be corrected by amending only a signal change-amount of the cycle where the error occurs, without amending all of the signal values of all cycles.

(d) The present method for detecting a target analyte comprising transforming a signal change-amount data set can provide a method for analyzing a target analyte by using various data such as a data set representing a change level of a signal-change amount data set or a reconstructed data set that is reconstructed by using the signal change-amount data set.

(e) The smoothing method of the present invention can reduce a noise of a data set simultaneously with baselining the data set. In addition, the degree of smoothing can be controlled by controlling the repeating number of data conversions.

The present invention will now be described in further detail by examples. It would be obvious to those skilled in the art that these examples are intended to be more concretely illustrative and the scope of the present invention as set forth in the appended claims is not limited to or by the examples.

### EXAMPLES

### EXAMPLE 1: Detection of Target Analyte by Using Cumulated Value of Signal Change Amount

In this Example, it was investigated whether a target analyte could be effectively detected through the present method which comprises reconstructing a data set obtained from a real-time PCR for the target nucleic acid by obtaining the cumulated values of the signal change amounts of the data set.

In order to perform the detection of a target analyte *(e.g.,* a target nucleic acid molecule), the process was used which comprises (i) providing a data set for a target analyte; (ii) providing a signal change-amount data set by acquiring signal change-amounts; (iii) providing a reconstructed data set by obtaining cumulated values of the signal change-amounts; and (iv) detecting the target analyte by using the reconstructed data set.

To evaluate the effect of the present method, the reconstructed data set was acquired by modifying the data set obtained from a real-time PCR for a target nucleic acid molecule and then the determination of the presence or absence of the target nucleic acid molecule was performed by using the acquired reconstructed data set with a predetermined target detection threshold.

### <1-1> Acquisition of Data Set

The real-time PCRs for a target nucleic acid molecule were performed using a probe as a signal-generating means with 50 cycles of an amplification on CFX96^{™} Real-Time PCR Detection System (Bio-Rad).

Among the data sets acquired from the real-time PCRs, three data sets were selected for the application of the present method. Figure 3 shows the amplification curves prepared by plotting the selected three data sets and were named as "data set 1, 2, and 3".

### <1-2> Acquisition of Signal Change-Amount Data Set

The signal change-amount data sets were obtained from the acquired raw data sets. Specifically, the signal change amounts were calculated by using "Difference Method" according to the following Equation 3. $Δ {y}_{i} = {y}_{i} - {y}_{i - 1} \left(if i=1,Δ{y}_{i}=0\right)$ Equation 3
i : a cycle number of a data set
*yᵢ*: a signal value at i^{th} cycle
Δyᵢ : the signal change-amount at i^{th} cycle

### <1-3> Baselining of Signal Change-Amount Data Set (Optional Step)

When the baseline of the data set was inclined as shown in the data set 2 or 3 of Figure 3, this baseline error was corrected through the amendment of the signal change-amount data set. On the contrary, when the baseline of the data set is not inclined as in the data set 1, this step of <1-3> does not need to be performed.

The specific value is subtracted from the signal change amounts at each cycle of the signal change-amount data set so that the baseline of the signal change-amount data set has a value of zero. Particularly, a specific cycle number or cycle region of an early reaction region *(i.e.,* the baseline region) in the signal change-amount data set, in which a signal is not substantially detected, is predesignated. After that, the average of the signal change amount in the predesignated specific cycle number or specific cycle region is calculated and the calculated average of the signal change amount is subtracted from the signal change amounts at each cycle.

In this Example, the average of the signal change amount for baselining the signal change-amount data set was calculated in the baseline region from the cycle number "3" (S cycle: start cycle) to the cycle number "10" (E cycle: end cycle) according to the following Equation 4. After that, the signal change amounts in the whole cycles of the signal change-amount data set were modified according to the following Equation 5 so that the average of the signal change amounts in the baseline region become zero . To identify the effectiveness of the baselining, the curve of signal change amounts (X axis: cycle number, Y axis: ΔRFU) was prepared by plotting the signal change-amount data set the baselining of which had been performed. $\overline{Δ y} = \frac{{\sum }_{i = S}^{E} {Δy}_{i}}{n}$ Equation 4
*i* : a cycle number of a data set
Δyᵢ: the signal change amount at i^{th} cycle
Δ̅y̅ : the average of signal change amounts in the baseline region
*S*: a start cycle number of baseline region in the signal change-amount data set
*E:* an end cycle number of baseline region in the signal change-amount data set
*n* : E - S + 1 $fit . Δ {y}_{i} = Δ {y}_{i} - \overline{Δ y}$ Equation 5
   i : a cycle number of a data set
   Δ*yᵢ* : the signal change amount at i^{th} cycle
   Δ̅*̅y̅*̅ : the average of signal change amounts in the baseline region
   *fit.*Δ*yᵢ* : the signal change amount at i^{th} cycle of the signal change-amount data set the baselining of which has been performed.

### <1-4> Acquisition of Reconstructed Data Set by Obtaining Cumulated Value of Signal Change Amount

The reconstructed data set was provided by obtaining the cumulative sums. The cumulative sums were acquired by cumulating signal change amounts from the first cycle up to the corresponding cycle at each cycle of a signal change-amount data set. In this Example, the cumulative sum was calculated by using the following Equation 6. The reconstructed amplification curve was prepared by plotting the reconstructed data set (X axis: cycle number, Y axis: RFU). $\begin{matrix}cum . {y}_{I} = CSV \left(CSC=I\right) \\ cum . {y}_{I} = CSV + {\sum }_{i = CSC + 1}^{I} Δ {y}_{i} \left(CSC<I\right) \\ cum . {y}_{I} = CSV + {\sum }_{i = I + 1}^{CSC} - Δ {y}_{i} \left(CSC>I\right)\end{matrix}$ Equation 6
*CSV* : the signal intensity at cumulation-starting cycle of the reconstructed data set
*CSC* : the cumulation-starting cycle number of a data set
*i* : a cycle number of a data set
*I* : a cycle number of which a cumulated value is to be calculated
Δyᵢ : the signal change amount at i^{th} cycle of a signal change-amount data set
*cum.yᵢ* : the signal intensity at i^{th} cycle of the reconstructed data set

Where the baselining has been performed on the signal change-amount data set, the value of *fit.*Δ*yᵢ* in Equation 5 would be the value of Δ*yᵢ* in Equation 6.

In this example, the "CSC" was designated as the value of "1", and the "CSV" was designated as the value of "0".

The curves, which were prepared by plotting the reconstructed data set obtained through the above steps, were compared each other.

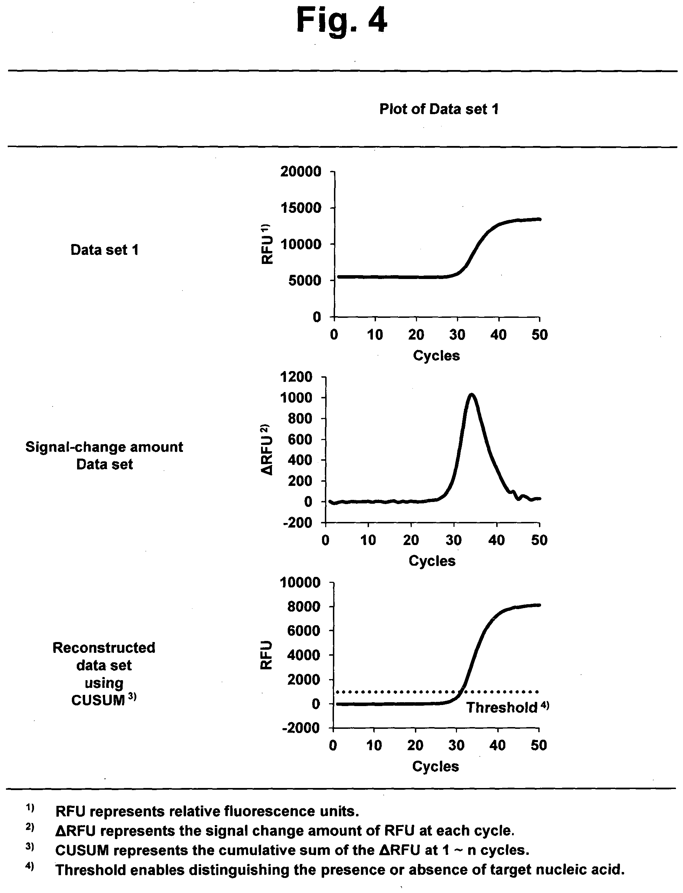

As shown in Figure 4 (data set 1), where the data set for a target analyte was processed through the steps of <1-1>, <1-2> and <1-4>, the data set was reconstructed in a manner that the signal values of the baseline region converged to zero. This result verifies that the process of the present method can make the signal values in the baseline region converge to zero or any desired value with no need of an establishment of the baseline region.

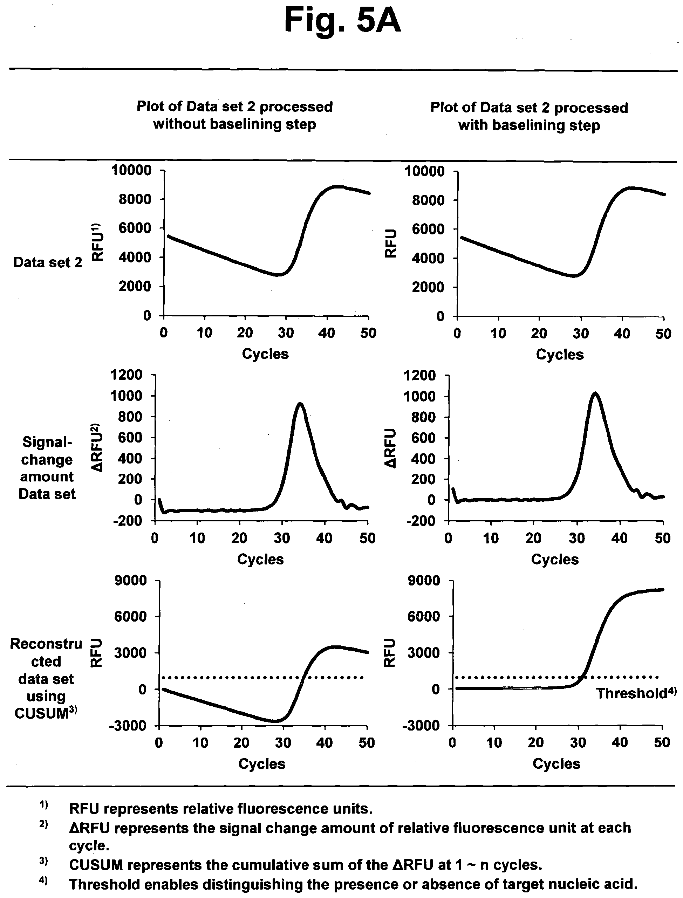

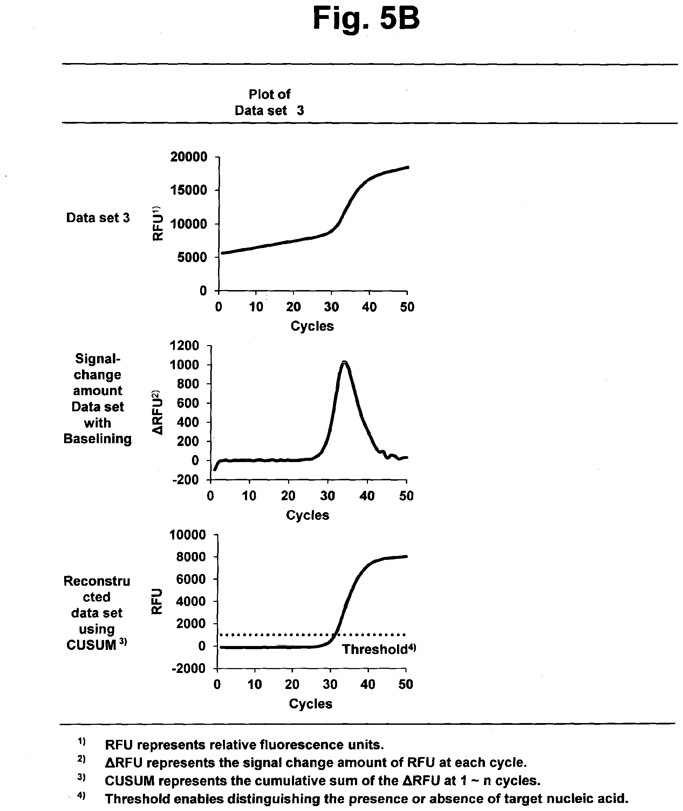

As shown in Figure 5A (data set 2), where the data set representing an inclined baseline was processed through the steps of <1-1>, <1-2> and <1-4> without baselining step, there was a problem that the inclined baseline could not be corrected even though the first signal value converged to zero. In that case, the addition of the step <1-3> between the steps <1-2> and <1-4> was proved to be capable of correcting the inclined baseline. (See the right column curves of Figure 5A). Moreover, the result shown in Figure 5B demonstrated that the inclined baseline of data set 3 could be also corrected by the additional application of the step <1-3>.

As a result, it would be understood that the inclined baseline of the signal change-amount data set could be successfully corrected by performing the additional step <1-3> (baselining step).

### <1-5> Detection of Target Analyte

The target analyte was detected by using the acquired reconstructed data set. The sample representing a fluorescence value over a predetermined threshold was determined as a positive and the sample exhibiting a fluorescence value below the predetermined threshold was determined as a negative. The predetermined threshold was designated as RFU 1000. As shown in "CUSUM" results of Figures 4, 5A, and 5B, it was proved that the determination of the target analyte detection could be successfully carried out by the present method without any detection error.

### EXAMPLE 2: Comparison of Various Calculation Methods for Obtaining Cumulated Value of Signal Change Amount

In this Example, the reconstructed data sets were obtained by using various methods for calculating cumulated values of the signal change amounts and the obtained reconstructed data sets were compared each other.

The reconstructed data set were prepared through the method comprising (i) providing a data set for a target analyte; (ii) providing a signal change-amount data set by acquiring signal change amounts; and (iii) providing a reconstructed data set by obtaining cumulated values of the signal change amounts.

### <2-1> Acquisition of Data Set

A data set 1 comprising fluorescence values (RFU) at each cycle was obtained from a real-time PCR as described in Example 1.

### <2-2> Acquisition of Signal Change-Amount Data Set

The signal change-amount data sets were obtained from the acquired raw data sets. Specifically, the signal change amounts were calculated by using "Difference Method" according to the above Equation 3, "Least Square Method" according to the following Equation 7, "Ratio Method" according to the following Equation 8 and "Differentiation" according to the following Differential Equation. $\begin{matrix}Δ {y}_{i} = \frac{{\sum }_{i = I - a}^{I + b} \left({x}_{i}-\overline{x}\right) \left({y}_{i}-\overline{y}\right)}{{\sum }_{i = I - a}^{I + b} {\left({x}_{i}-\overline{x}\right)}^{2}} \\ \overline{x} = \frac{{\sum }_{i = I - a}^{I + b} {x}_{i}}{n} , \overline{y} = \frac{{\sum }_{i = I - a}^{I + b} {y}_{i}}{n}\end{matrix}$ Equation 7
*I* : a cycle number of a data set of which a signal change amount is to be calculated
*xᵢ* : a cycle number of i^{th} cycle
*yᵢ* : a signal intensity measured at i^{th} cycle
Δ*yᵢ* : the signal change amount at i^{th} cycle
"a" and "b" : an integer from 0 to 10
*n* : a + b + 1, a number of data that is used to calculate a signal change amount
x̅: the average of cycle numbers from "I-a" to "I+b"
y̅: the average of signal intensities measured at cycles from "I-a" to "I+b"

In this Example, "1" is used for "a" and "b". For data points at which "Ia" is less than "1", the "a" may be altered to permit "I-a" to become "1". For data points at which *"I+b"* is more than the number of all data points, the "b" may be altered to permit *"I+b"* to be equal to the number of all data points. ${Δy}_{i} = \frac{{y}_{i}}{{y}_{i - 1}} \left(if i=1, Δ{y}_{i}=1\right)$ Equation 8
*i* : a cycle number of a data set
*yᵢ* : a signal intensity measured at i^{th} cycle
Δyᵢ : the signal change amount at i^{th} cycle $Δ {y}_{i} = 4 . E - 04 {{x}_{i}}^{5} - 0.0547 {{x}_{i}}^{4} + 2.6164 {{x}_{i}}^{3} - 52.906 {{x}_{i}}^{2} + 433.63 {x}_{i} - 1072.3$ Differential Equation
   *i* : a cycle number of a data set
   *xᵢ* : a cycle number of i^{th} cycle
   Δyᵢ : the signal change amount at i^{th} cycle

The above Differential Equation was obtained by differentiation of the six order polynomial calculated from the raw data set.

### <2-3> Acquisition of Reconstructed Data Set by Obtaining Cumulated Value of Signal Change Amount

The reconstructed data sets were provided by obtaining cumulated values of signal change amounts, *i.e.,* the cumulative sums, the cumulative products and the integration of the signal change-amounts. Specifically, the cumulated values were calculated by using "Cumulative Sum" according to the above Equation 6, "Cumulative Product" according to the following Equation 9 and "Integration" according to the following Integral Equation. $\begin{matrix}cum . {y}_{I} = CSV \left(CSC=I,CSV\neq 0\right) \\ cum . {y}_{I} = CSV \times {\prod }_{i = CSC + 1}^{I} Δ {y}_{i} \left(CSC<I\right) \\ cum . {y}_{I} = CSV \times {\prod }_{i = I + 1}^{CSC} \frac{1}{Δ {y}_{i}} \left(CSC>I\right)\end{matrix}$ Equation 9
*CSV* : the signal intensity at cumulation-starting cycle of the reconstructed data set
*CSC* : the cumulation-starting cycle number of a data set
*I* : a cycle number of which a cumulated value is to be calculated
*i* : a cycle number of a data set
Δ*yᵢ* : the signal change amount at i^{th} cycle of a signal change-amount data set *cum.yᵢ* : the signal intensity at i^{th} cycle of the reconstructed data set $\begin{matrix}cum . Δ {y}_{i} = 3 E - 06 {{x}_{i}}^{7} - 0.0004 {{x}_{i}}^{6} + 0.0237 {{x}_{i}}^{5} - 0.6453 {{x}_{i}}^{4} + 8.2817 {{x}_{i}}^{3} - 45.475 {{x}_{i}}^{2} \\ + 75.669 {x}_{i}\end{matrix}$ Integral Equation
   *i* : a cycle number of a data set
   *xᵢ* : a cycle number of i^{th} cycle
   *cum.yᵢ* : the signal intensity at i^{th} cycle of the reconstructed data set

The above Integral Equation was obtained by integration of the six order polynomial calculated from the signal change-amount data set.

The curves, which were prepared by plotting the reconstructed data set obtained through the above steps, were compared each other.

### <2-4> Comparison of Reconstructed Data Sets Obtained by Using Various Cumulation-Starting Cycles and Cumulation-Starting Values

The reconstructed data sets were obtained from the signal change-amount data set by using various cumulation-starting cycles (CSCs) and cumulation-starting values (CSVs) and the obtained reconstructed data sets were compared each other.

The signal change-amount data sets were obtained by using "Difference Method" according to the above Equation 3, and the reconstructed data sets were obtained by using "Cumulative Sum" according to the above Equation 6. "CSC" was designated as the values of "1", "34" and "50" respectively, and "CSV" was designated as the values of "-2000", "0" and "2000" respectively.

The subtracted data sets were obtained from the reconstructed data set by subtracting a certain value from the signal values at each cycle of the reconstructed data set in a manner that the signal value at the cycle number "1" is converged to zero.

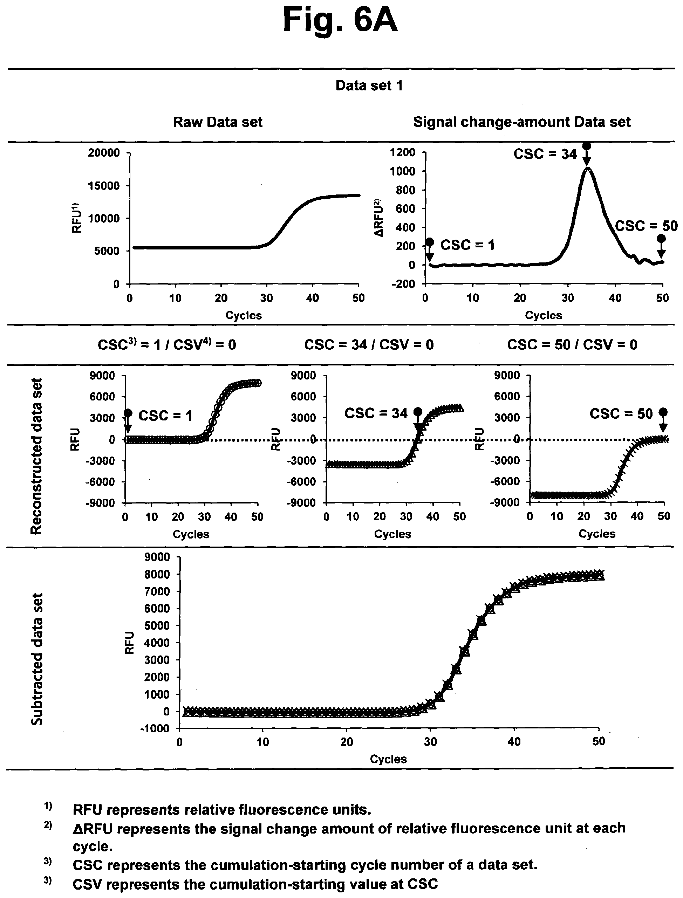

As shown in Fig. 6A, the individual reconstructed data sets have different signal values at the same cycle when the data sets were obtained by using different cumulation-starting cycles, but have the same signal value *(i.e.,* the same cumulation-starting value) at the cumulation-starting cycle because the same cumulation-starting value were used. However, the respective subtracted data sets have the same signal value at the same cycle regardless of the difference of the "cumulation-starting cycle".

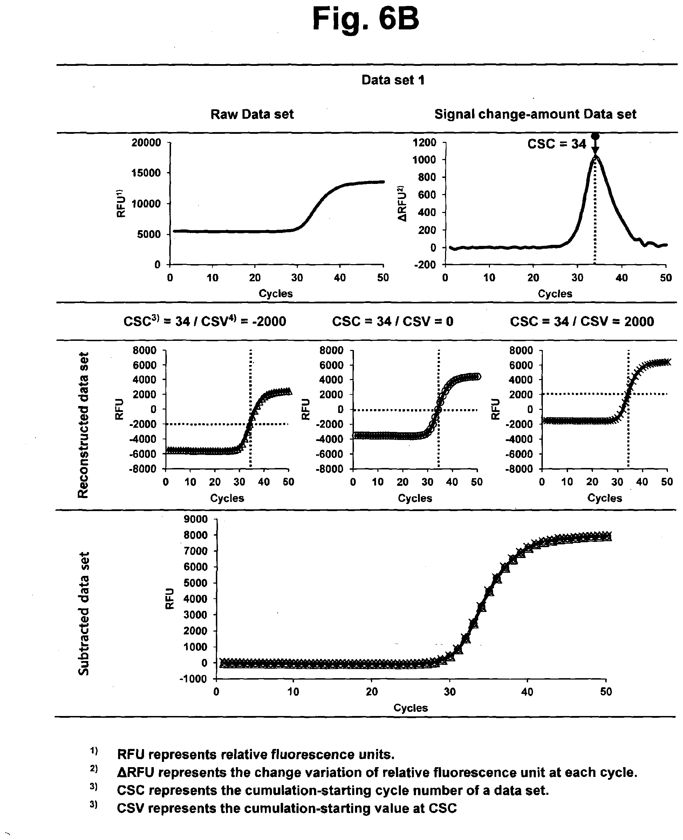

As shown in Fig. 6B, the individual reconstructed data sets have different signal values at the same cycle when the data sets were obtained by using different cumulation-starting values, but have the same signal value as the cumulation-starting value. However, the respective subtracted data sets have the same signal value at the same cycle regardless of the difference of the "cumulation-starting value".

As a result, it would be demonstrated that the respective reconstructed data sets have the same signal pattern even though the signal intensities of the cumulated value *(i.e.,* Y-axis value) calculated using different cumulation-starting cycles or cumulation-starting values were different from each other.

### <2-5> Comparison of Various Methods for Calculation of the Signal Change Amount and Cumulated Value

The reconstructed data sets were obtained by using various methods for calculating the signal change amounts and cumulated values and the obtained reconstructed data set were compared with each other.

The signal change-amount data sets were obtained by using "Least Square Method" according to the above Equation 7, "Ratio Method" according to the above Equation 8 and "Differentiation Method" according to the above Differential Equation.

The reconstructed data sets were obtained by using "Cumulative Sum" according to the above Equation 6, "Cumulative Product" according to the above Equation 9 and "Integration" according to the above Integral Equation.

When the reconstructed data set was obtained by using "Cumulative Sum", the "CSC" was designated as the value of "1", and the "CSV" was designated as the value of "0". In addition when the reconstructed data set was obtained by using "Cumulative Product", the "CSC" was designated as the value of "1", and the "CSV" was designated as the value of "1.00".

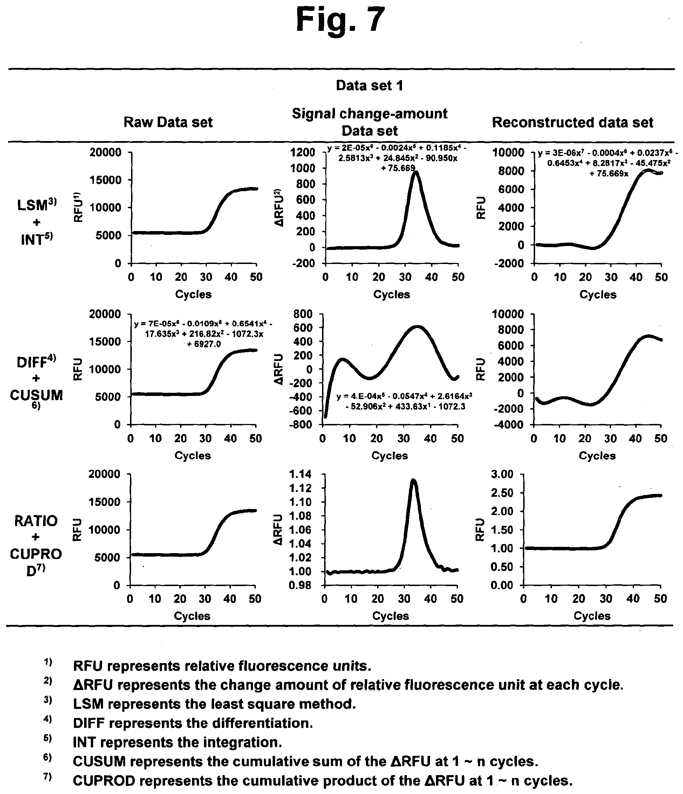

As shown in Fig. 7, it was proved that the signal change-amount data set could be obtained by various methods for calculating the signal change amount from the raw data set. In addition, the reconstructed data set could be obtained by various methods for calculating the cumulated value from the signal change-amount data set.

As a result, it would be understood that the various known calculation methods can be used for the calculation of the signal change amount and cumulated value.

### EXAMPLE 3: Correction of Detection Error by Amending Signal Change-Amount

In Example 3, it was investigated whether an error in determining the presence or absence of a target analyte caused by an abnormal signal of a data set could be removed by the present method using an amendment of signal change amounts.

In this Example, the method of reconstructing a data set with amendment of signal change amounts was performed by the process comprising (i) providing a data set for a target analyte; (ii) providing a signal change-amount data set by obtaining signal change amounts; (iii) correcting an abnormal signal in the obtained signal change-amount data set; (iv) providing a reconstructed data set by obtaining cumulated values of the signal change amounts; and (v) detecting the target analyte by using the reconstructed data set.

### <3-1> Acquisition of Data Set Including Abnormal Signal

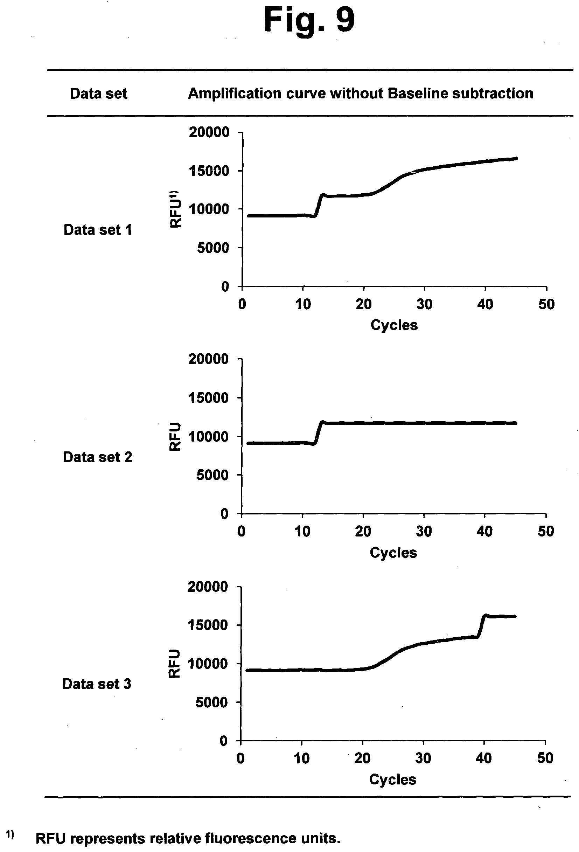

The real-time PCRs for a target nucleic acid molecule were performed using probe as a signal-generating means with 50 cycles of an amplification on CFX96^{™} Real-Time PCR Detection System (Bio-Rad). Among the data sets obtained from the real-time PCRs, three data sets including Jump error *(i.e.,* abnormal signal) were selected for the application of the present method. Figure 9 illustrates the amplification curves prepared by plotting the selected three data sets.

### <3-2> Acquisition of Signal Change-Amount Data Set

The signal change-amount data sets were obtained from the raw data sets. Specifically, the signal change-amounts were calculated by using "Least Square Method" according to the above Equation 7 and example <2-2>.

### <3-3> Correction of Abnormal Signal in Signal Change-Amount Data Set

The correction of an abnormal signal of signal change-amount data set is performed by the processes comprising (a) detecting an abnormal signal and (b) correcting an abnormal signal.

### a. Detecting Abnormal Signal

The "threshold for signal change amount" for determining the presence or absence of a signal change-amount peak was designated as a value of "200". Where a cycle representing a signal change-amount over 200 is found in a signal change-amount data set, it can be determined that the signal change-amount peak is present. After determining the presence of a signal change-amount peak, the method of "Half Peak Width" is used to identify an abnormal peak from a normal peak. The calculation of "Half Peak Width" was performed according to the following Equation 10. $Half Peak Width \left(=Δcycle\right) = Max cycle - Start cycle$ Equation 10

Max cycle: a cycle number of the data point that represents the maximum signal change amount within a peak.

Start cycle: a cycle number of a data point that represents firstly a signal change amount over "threshold for signal change-amount" before the max cycle within a peak.

After calculating the "Half Peak Width", an abnormal peak *(i.e.,* an abnormal signal) was identified from a normal peak by using a predesignated "threshold for peak". In this Example, the "threshold for peak" was designated as a value of "2". Where the calculated "Half Peak Width" is less than the "threshold for peak", the peak is determined to be an abnormal peak. Contrary to this, where the "Half Peak Width" is over the predesignated "threshold for peak", the peak is determined to be a normal peak.

### b. Correcting Abnormal Signal

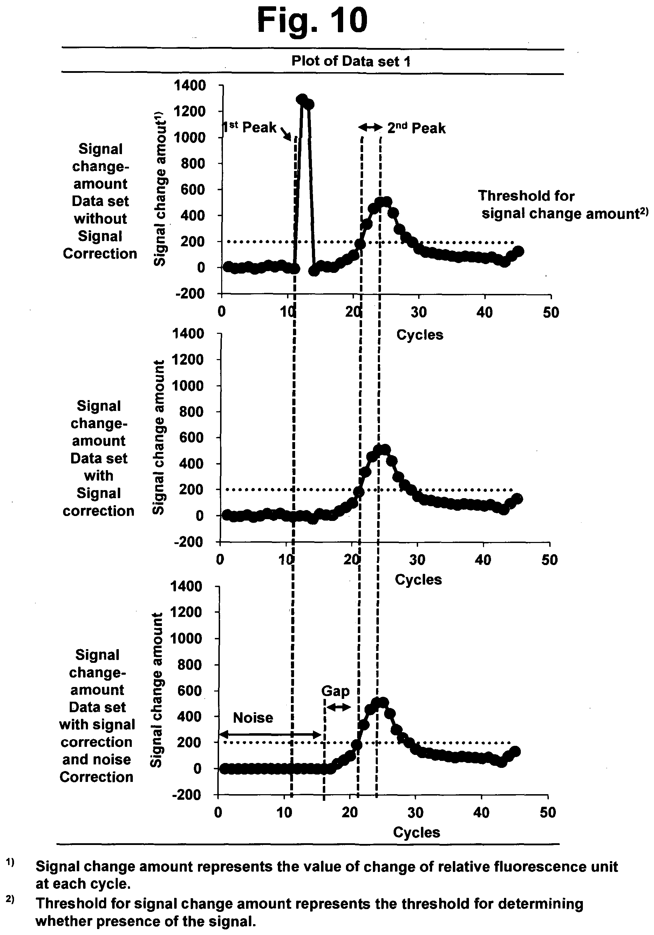

After identifying an abnormal peak, the peak representing the abnormal signal was corrected. Specifically, as shown in the second curve of Figure 10, the portion of signal change-amounts that was over the "threshold for signal change-amount" were corrected to zero and thereby providing an amended signal change-amount data set.

### c. Noise Correction (Optional Step)

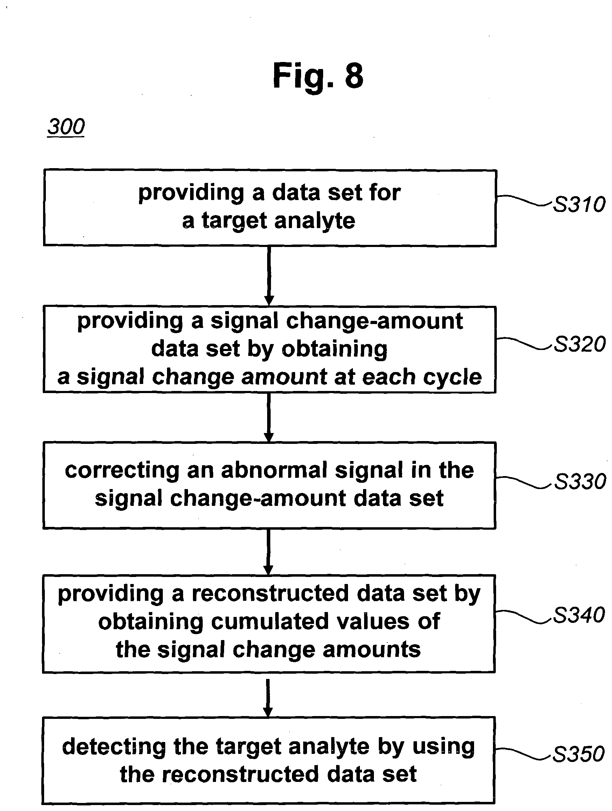

This process was optionally used to correct a background noise signal after correcting an abnormal peak. Particularly, as shown in the third curve of Figure 8, where a normal peak was present after removing the abnormal peak, the signal change-amounts from the "0" cycle to the cycle before the start of the normal peak were made to zero. Moreover, where a normal peak was absent, all signal peaks at every cycle were recognized as noises and the signal change amounts were corrected to zero. The cycle number immediate before the start of the normal peak was determined by subtracting the predetermined cycle number ("Gap") from the cycle number that represents a crossing point between the curve and threshold for signal change-amount.

### <3-4> Acquisition of Reconstructed Data Set by Cumulating Signal Change Amount and Detection of Target Analyte

The reconstructed data set was acquired by cumulating signal change amounts from the amended signal change-amount data set and then the target nucleic acid was detected by using the reconstructed data set. The acquisition of the reconstructed data set and the detection of the target nucleic acid were performed according to the same method as described in Example 1. In this Example, the threshold for target detection was designated as RFU 500.

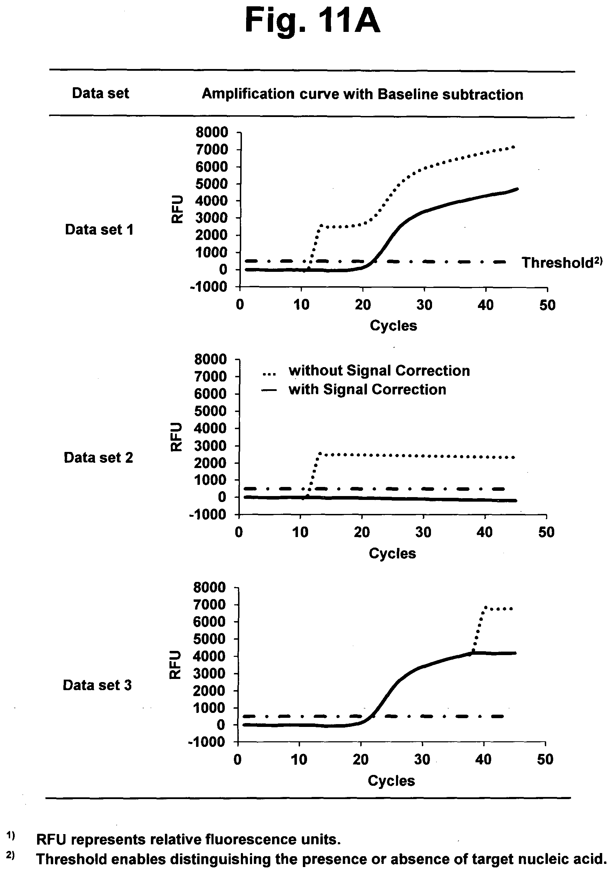

The reconstructed data sets that had been obtained through amendment *(i.e., a* correction of an abnormal signal) and cumulation of signal change amounts in the signal change-amount data sets were plotted in Figure 11A. For comparison, the reconstructed data sets obtained by cumulating signal change-amounts of the signal change-amount data sets without amendment were also shown with dotted line curve in Figure 11A.

As shown in Figure 11A, the abnormal signals (e.g., jumping error) were corrected by amendment of the signal change amounts. Moreover, the abnormal signals could be solely corrected without affecting normal signals even if the normal and abnormal signals coexisted. For data sets 1 and 3, it was determined that target nucleic acid molecules were detected without an error. In addition, for data set 2 containing no target nucleic acid molecule, it was also correctly determined that no target nucleic acid molecule was detected.

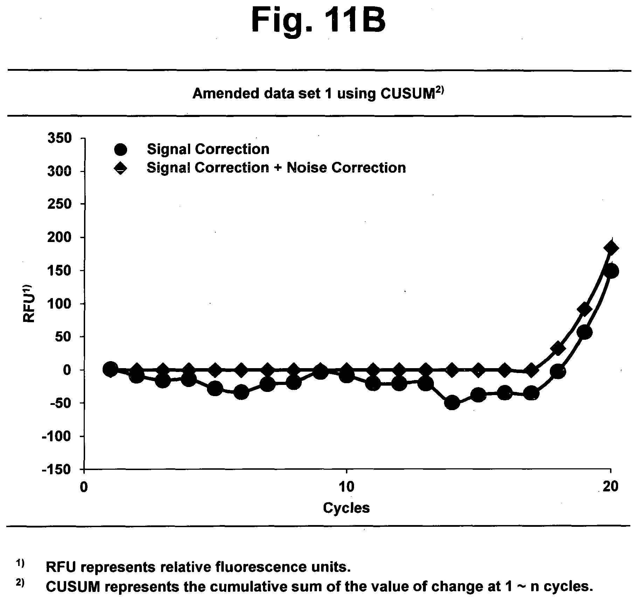

To identify the effectiveness of the noise-correcting process, the amended signal change-amount data set with a noise correction and the change-level data set without a noise correction were plotted respectively. As shown in Figure 11B which depicts a region of cycles including a noise signal of the amended / non- amended change-level data set 1, the noise signals have been removed and the signal level became constant without a variation in the amended data set 1.

From the above results, it could be understood that the method of the present disclosure is able to correct an abnormal signal in the amplification curve and thereby effectively reducing an error in determining the presence or absence of the target nucleic acid that is caused by an abnormal signal. Moreover, the present method could provide a modified data set that is capable of more accurately detecting the amount of a target analyte.

### EXAMPLE 4: Detection of Target Analyte by Using Transformed Data Set

In Example 4, it was investigated whether a target analyte can be detected by a process comprising amending a signal change-amount data set for the target analyte and transforming the amended signal change-amount data set.

In this Example, the method of detecting a target analyte was performed by the process comprising (i) providing a data set for a target analyte; (ii) providing a signal change-amount data set by obtaining signal change amounts and amending the obtained signal change-amount data set; (iii) transforming the amended signal change-amount data set; and (iv) detecting the target analyte by using the transformed data set.

### <4-1> Acquisition of Data Set

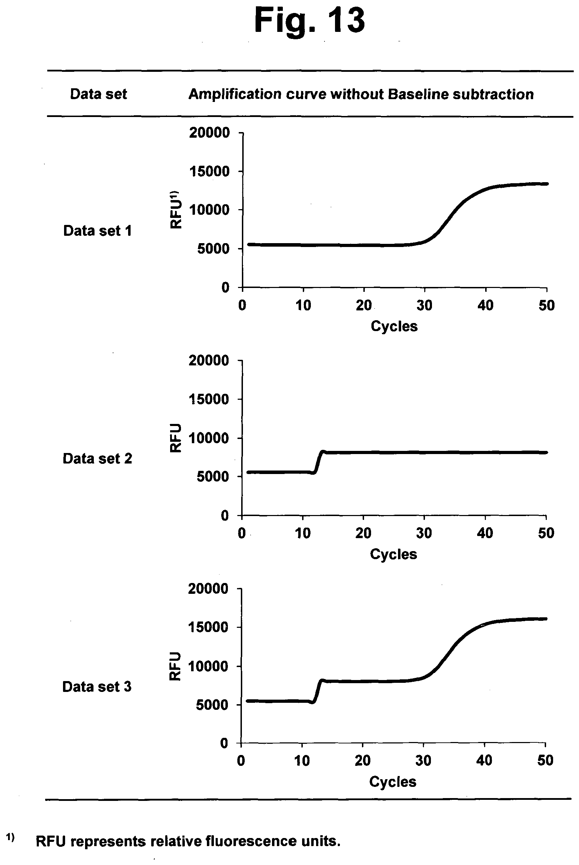

The real-time PCRs for a target nucleic acid molecule were performed using a probe as a signal-generating means with 50 cycles of an amplification on CFX96^{™} Real-Time PCR Detection System (Bio-Rad). Among the data sets obtained from the real-time PCRs, three data sets were selected for the application of the present method. Figure 13 illustrates the amplification curves prepared by plotting the three data sets.

### <4-2> Acquisition and Amendment of Signal Change-Amount Data Set

The signal change-amount data sets were obtained from the three raw data sets by calculating the first order signal change amounts. After that, abnormal signals were removed by amending the first order signal change amounts in the signal change-amount data sets.

The acquisition and further amendment of the signal change-amount data sets were performed according to the same method as described in Examples <3-2> and <3-3>. The correction of abnormal signals was also performed by the method described in Example <3-3>. Specifically, after detecting an abnormal signal, the only portion of cycles in a peak that includes the signal change amount over the value of 200 *(i.e.,* threshold for signal change amount) in the signal change-amount data set was corrected to zero.

### <4-3> Transformation of Amended Signal Change-Amount Data Set

The transformed data set was obtained by transforming the amended signal change-amount data set and the target analyte was detected by using the transformed data set. In this Example, the transformation was performed by acquiring the 2^{nd} order signal change amounts from the signal change-amount data set.

Specifically, the 2^{nd} order signal change amounts were calculated by using the following Equation 11. For the detection of the target analyte, the curve of the transformed data set was prepared by plotting the 2^{nd} order signal change-amount data set. $\begin{matrix}cor . {ss}_{i} = \frac{{\sum }_{i = I - a}^{I + b} \left({x}_{i}-\overline{x}\right) \left(cor.{s}_{i}-\overline{cor . {s}_{i}}\right)}{{\sum }_{i = I - a}^{I + b} {\left({x}_{i}-\overline{x}\right)}^{2}} \\ \overline{x} = \frac{{\sum }_{i = I - a}^{I + b} {x}_{i}}{n} , \overline{cor . {s}_{i}} = \frac{{\sum }_{i = I - a}^{I + b} cor . {s}_{i}}{n}\end{matrix}$ Equation 11
I : a cycle number of a data set of which a signal change-amount is to be calculated
*xᵢ* : a cycle number of i^{th} cycle
*cor.sᵢ* : an amended signal change amount at i^{th} cycle
*cor.ssᵢ* :a transformed signal change amount at i^{th} cycle
"a" and "b" : an integer from 0 to 10
*n :* a + b + 1, a number of data used to calculate a signal change amount
x̅: the average of cycle numbers from "I-a" to "I+b"
y̅: the average of amended signal change-amounts at cycles from "I-a" to "I+b"

In this Example, "1" is used for "a" and "b". For data points at which "*I*-*a*" is less than "1", the "a" may be altered to permit "I-a" to become "1". For data points at which *"I+b"* is more than the number of all data points, the "b" may be altered to permit *"I+b"* to be equal to the number of all data points.

### <4-4> Detection of Target Analyte

The target nucleic acid molecule *(i.e.,* the target analyte) was detected by using the transformed data set. The sample representing a 2^{nd} order signal change amount over a predetermined threshold was determined as a positive and the sample showing a 2^{nd} order signal change amount below the predetermined threshold was determined as a negative. The predetermined threshold for the target detection was designated as the value "70" in the 2^{nd} order signal change-amount data set in this Example.

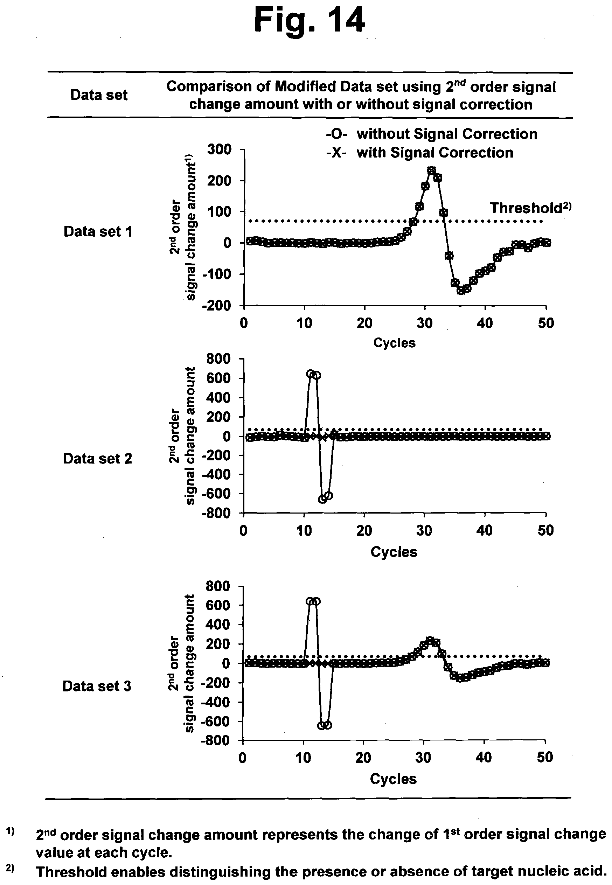

As shown in Figure 14 depicting the curves of the transformed data set, the results of determining the detection of a target analyte was different depending on whether amendment on signal change-amounts had been applied or not.

Specifically, in the positive sample containing only normal signals *(i.e.,* data set 1), the same results for determining the presence of the target nucleic acid were observed regardless of the application of amendment on signal change amounts.

Further, in the negative sample containing abnormal signals *(i.e.,* data set 2), where the signal change amounts were not amended, a false positive result was observed because the abnormal signal was over the threshold for target detection. On the contrary, where the signal change amounts were amended, the true negative result was observed because the abnormal signals were removed in the curves of the transformed data sets.

Moreover, in the positive sample containing abnormal signals *(i.e.,* data set 3), where the signal change amounts were not amended, a detection error was occurred because the abnormal signal was also over the threshold for target detection. On the contrary, where the signal change amounts were amended, the true positive result was observed because the abnormal signals were removed the curves of the transformed data sets.

As a result, it would be verified that the present method comprising amendment and the transformation of the signal change-amount data set could get rid of the detection error for a target analyte and thus enable us to detect accurately the target analyte.

### EXAMPLE 5: Method for Smoothing Data Set by Using Reconstruction of Data Set

In Example 5, it was investigated whether the curves prepared by plotting the reconstructed data set could become smoothed.

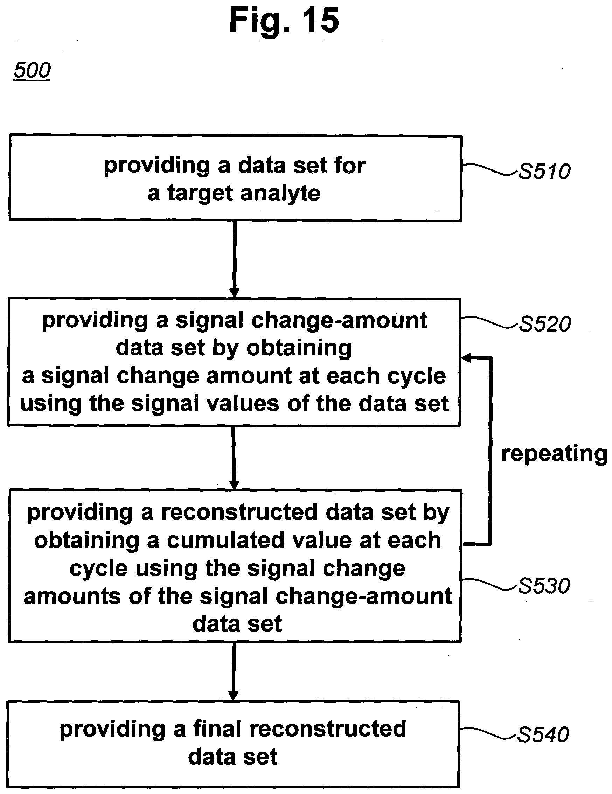

As shown in Figure 15, the method for smoothing a data set was performed by a processes comprising (i) providing a data set for a target analyte; (ii) providing a signal change-amount data set by obtaining signal change amount at each cycle using the signal values of the data set; and (iii) providing a reconstructed data set by obtaining cumulated value at each cycle using the signal change amounts of the signal change-amount data set. In addition, the process optionally could comprise a repetition of the steps (ii) and (iii).

### <5-1> Acquisition of Data Set

A data set comprising fluorescence values (RFU) at each cycle was obtained from a real-time PCR as described in Example 1.

### <5-2> Acquisition of Signal Change-Amount Data Set

A signal change-amount data set was obtained from the acquired data set by obtaining signal change amounts. The signal change amounts were calculated by using "Least Square Method" according to the above Equation 7.

In this Example, "2" is used for "a" and "b". For data points at which "I-a" is less than "1", the "a" may be altered to permit "I-a" to become "1". For data points at which "I+b" is more than the number of all data points, the "b" may be altered to permit "I+b" to be equal to the number of all data points.

The baselining was performed on the signal change-amount data set at one time before the first reconstruction of the signal change-amount data set according to the same method as described in Example <1-3>.

### <5-3> Acquisition of Reconstructed Data Set by Obtaining Cumulated Value of Signal Change Amount

The reconstructed data set was prepared by obtaining the cumulative sums at each cycle of the signal change-amount data set. The cumulative sums were calculated by using the above Equation 6.

In this example, "CSC" was designated as the value of "1", and "CSV" was designated as the value of "0".

### <5-4> Improving Smoothing Effect by Repeating Reconstruction of Data Set

It was further investigated whether the effect of smoothing data set could be improved by repeating the process of reconstructing the data set. The final reconstructed data set was obtained by repeating the steps <5-2> and <5-3> in which the reconstructed data set obtained in the step <5-3> was reused as the data set for the step <5-2>. Specifically, the repetition of the steps <5-2> and <5-3> was performed two times and thus a total of three reconstructed data sets were obtained. When repeating the reconstruction of the data set, the values "three (3)" and "one (1)" were respectively used for "a" and "b" in Equation 7 in order to prevent the alteration of the signal- generating point.

For identifying the improvement of the data set smoothing-effect, all of the reconstructed data sets were plotted and analyzed with comparing them.

Moreover, the detection of target nucleic acid was performed by using the reconstructed data sets at each step of the repetition of the reconstructions. The sample representing a fluorescence value over the predetermined threshold for target detection was determined as a positive and the sample showing a fluorescence value below the predetermined threshold was determined as a negative. In this Example, the predetermined threshold for the target detection was designated as RFU 300.

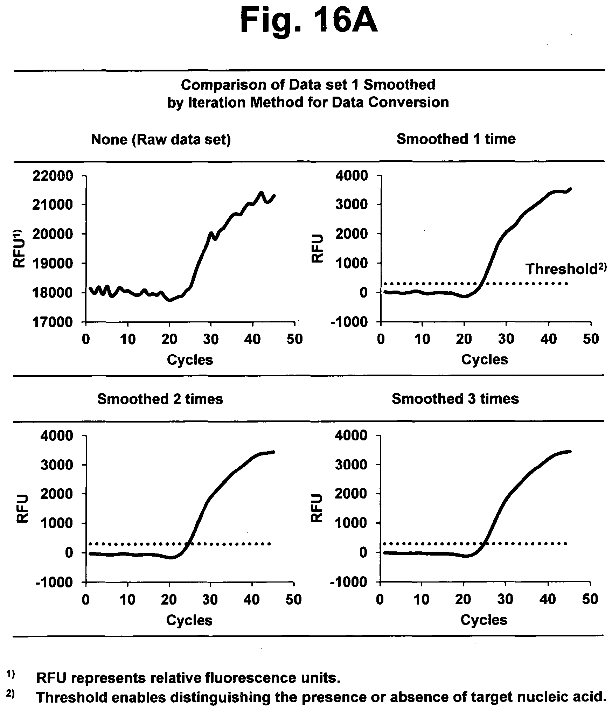

As shown in Figure 16A, the smoothing effect for the data set was exhibited in the first reconstructed data set and was also more improved with the increase of the repetition of the reconstruction process. In addition, there was no error in determining the target nucleic acid detection.

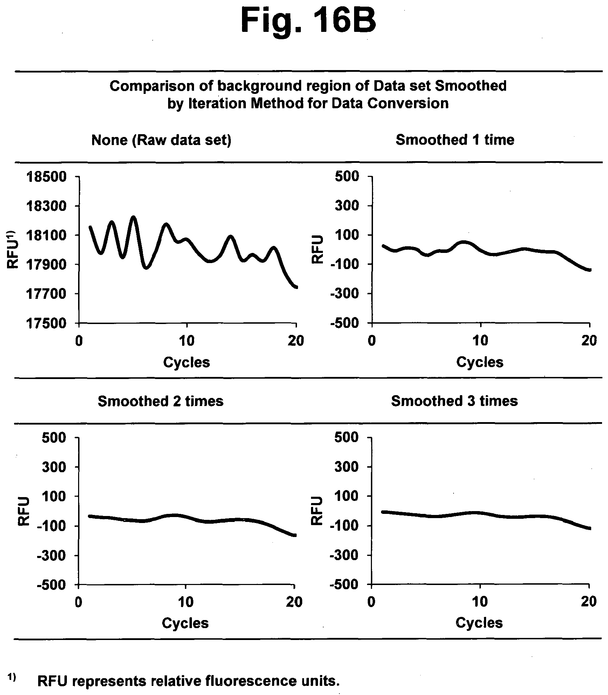

Figure 16B illustrates the enlarged curves depicting the portion of cycles from 0 to 20 corresponding to the background region of the reconstructed data sets. As shown in Figure 16B, the smoothing-effect for the data set was manifested in the background region *(i.e.,* cycles from 0 to 20).

It would be addressed that the present method of reconstructing a data set could smooth the data set-plotted curve thereby removing a noise signal in the background region. Therefore, the present method can ensure the accurate and reliable detection and quantification of a target analyte in the sample.

Having described a preferred embodiment of the present invention, it is to be understood that variants and modifications thereof falling within the scope of the invention may become apparent to those skilled in this art, and the scope of this invention is to be determined by appended claims and their equivalents.

## Claims

1. A method for detecting a target analyte in a sample, comprising:
- (a) providing a data set for the target analyte; wherein the data set is obtained from a signal-generating process for the target analyte using a signal-generating means; wherein the data set comprises a plurality of data points, each data point comprising a cycle and a signal value at the cycle;
- (b) providing a signal change-amount data set by obtaining a signal change amount at each cycle using the signal values of the data set; wherein the signal change-amount data set comprises a plurality of data points comprising the cycles and signal change-amounts at the cycles;
- (c) providing a reconstructed data set by obtaining a cumulated value at each cycle using the signal change amounts of the signal change-amount data set; wherein the reconstructed data set comprises a plurality of data points comprising cycles and cumulated values at the cycles; and
- (d) detecting the target analyte in the sample by using the reconstructed data set.

2. The method according to claim 1, wherein the cumulated value at each cycle is calculated by one of the following calculations depending on the number of said each cycle (Xᵢ) relative to the number of a cumulation-starting cycle (CSC):
wherein the cumulation-starting cycle (CSC) is a cycle selected from the cycles of the signal change-amount data set;
- (Cal-1) wherein when Xᵢ is larger than the number of CSC, the cumulated value at said each cycle is calculated by cumulating (i) a cumulation-starting value and (ii) signal change amount(s) from a cycle immediately following the cumulation-starting cycle to said each cycle; wherein the cumulation-starting value is a cumulated value at the cumulation-starting cycle;
- (Cal-2) wherein when Xᵢ is smaller than the number of CSC, the cumulated value at said each cycle is calculated by cumulating (i) the cumulation-starting value and (ii) a value(s) derived from signal change amount(s) from a cycle immediately following said each cycle to the cumulation-starting cycle; and
- (Cal-3) wherein when Xᵢ is equal to the number of CSC, the cumulation-starting value is designated as the cumulated value at said each cycle.

3. The method according to claim 1, wherein the signal change amount at each cycle is selected from the group consisting of a derivative value of the signal values at each cycle; a difference in the signal values at each cycle; a ratio of the signal values at each cycle; and a slope value obtained by performing a linear regression analysis at each cycle.

4. The method according to claim 1, wherein the cumulated value at each cycle is selected from the group consisting of a cumulated value of derivative values at each cycle; a cumulated value of differences at each cycle; a cumulated value of ratios at each cycle; and a cumulated value of slope values.

5. The method according to claim 1, wherein the method further comprises the step of:
modifying at least one signal change-amount(s) of the signal change-amount data set.

6. The method according to claim 1, wherein the data set of the step (a) is a raw data set, a mathematically modified data set of the raw data set, a normalized data set of the raw data set or a normalized data set of the modified data set of the raw data set.

7. The method according to claim 1, wherein the signal change-amount data set is a baseline subtracted signal change-amount data set.

8. The method according to claim 1, wherein the signal change amount is obtained by a linear regression analysis method.

9. The method according to claim 1 and 8, wherein the method further comprises the step of repeating the steps (b)-(c) for smoothing the data set in which the reconstructed data set provided by the step (c) is used as the data set for the step (b).

10. A computer readable storage medium containing instructions to configure a processor to perform a method for detecting a target analyte in a sample, the method comprising:
- (a) receiving a data set for the target analyte;
wherein the data set is obtained from a signal-generating process for the target analyte using a signal-generating means; wherein the data set comprises a plurality of data points, each data point comprising a cycle and a signal value at the cycle;
- (b) providing a signal change-amount data set by obtaining a signal change amount at each cycle using the signal values of the data set; wherein the signal change-amount data set comprises a plurality of data points comprising the cycles and signal change-amounts at the cycles;
- (c) providing a reconstructed data set by obtaining a cumulated value at each cycle using the signal change amounts of the signal change-amount data set; wherein the reconstructed data set comprises a plurality of data points comprising cycles and cumulated values at the cycle; and
- (d) determining the presence or absence of the target analyte in the sample by using the reconstructed data set.

## Patentansprüche

1. Verfahren zum Nachweis eines Zielanalyten in einer Probe, umfassend:
- (a) Bereitstellen eines Datensatzes für den Zielanalyten, wobei der Datensatz aus einem Signalerzeugungsprozess für den Zielanalyten unter Verwendung eines Signalerzeugungsmittels erlangt wird, wobei der Datensatz eine Vielzahl von Datenpunkten umfasst, wobei jeder Datenpunkt einen Zyklus und einen Signalwert beim Zyklus umfasst;
- (b) Bereitstellen eines Datensatzes mit Signalwechselmenge, indem bei jedem Zyklus unter Verwendung der Signalwerte des Datensatzes eine Signalwechselmenge erlangt wird, wobei der Datensatz mit Signalwechselmenge eine Vielzahl von Datenpunkten umfasst, die die Zyklen und die Signalwechselmengen bei den Zyklen umfassen;
- (c) Bereitstellen eines rekonstruierten Datensatzes durch Erlangen eines kumulierten Werts bei jedem Zyklus unter Verwendung der Signalwechselmengen des Datensatzes mit Signalwechselmenge, wobei der rekonstruierte Datensatz eine Vielzahl von Datenpunkten umfasst, die Zyklen und kumulierte Werte bei den Zyklen umfassen; und
- (d) Nachweisen des Zielanalyten in der Probe unter Verwendung des rekonstruierten Datensatzes.

2. Verfahren nach Anspruch 1, wobei der kumulierte Wert bei jedem Zyklus anhand einer der folgenden Berechnungen berechnet wird, abhängig von der Nummer des jeweiligen Zyklus (Xᵢ) im Verhältnis zur Nummer des Kumulierungsstartzyklus (cumulation-starting cycle; CSC):
wobei der Kumulierungsstartzyklus (CSC) ein Zyklus ist, der aus den Zyklen des Datensatzes mit Signalwechselmenge ausgewählt wird;
- (Cal-1), wobei, wenn Xᵢ größer ist als die Nummer des CSC, der kumulierte Wert bei dem jeweiligen Zyklus berechnet wird, indem (i) ein Kumulierungsstartwert und (ii) eine oder mehrere Signalwechselmengen von einem Zyklus, der unmittelbar auf den Kumulierungsstartzyklus folgt, bis zu dem jeweiligen Zyklus kumuliert werden, wobei der Kumulierungsstartwert ein kumulierter Wert bei dem Kumulierungsstartzyklus ist;
- (Cal-2), wobei, wenn Xᵢ kleiner ist als die Nummer des CSC, der kumulierte Wert bei dem jeweiligen Zyklus berechnet wird, indem (i) der Kumulierungsstartwert und (ii) ein oder mehrere Werte, die aus einer oder mehreren Signalwechselmengen von einem unmittelbar auf den jeweiligen Zyklus folgenden Zyklus bis zu dem Kumulierungsstartzyklus abgeleitet werden, kumuliert werden; und
- (Cal-3), wobei, wenn Xᵢ der Nummer des CSC entspricht, der Kumulierungsstartwert als kumulierter Wert bei dem jeweiligen Zyklus festgelegt wird.

3. Verfahren nach Anspruch 1, wobei die Signalwechselmenge bei jedem Zyklus aus der Gruppe ausgewählt wird, die aus einem Ableitungswert der Signalwerte bei jedem Zyklus, einer Differenz der Signalwerte bei jedem Zyklus, einem Verhältnis der Signalwerte bei jedem Zyklus und einem Steigungswert, der durch Durchführung einer linearen Regressionsanalyse bei jedem Zyklus erlangt wird, besteht.

4. Verfahren nach Anspruch 1, wobei der kumulierte Wert bei jedem Zyklus aus der Gruppe ausgewählt wird, die aus einem kumulierten Wert von Ableitungswerten bei jedem Zyklus, einem kumulierten Wert von Differenzen bei jedem Zyklus, einem kumulierten Wert von Verhältnissen bei jedem Zyklus und einem kumulierten Wert von Steigungswerten besteht.

5. Verfahren nach Anspruch 1, wobei das Verfahren ferner den folgenden Schritt umfasst:
Modifizieren mindestens einer Signalwechselmenge des Datensatzes mit Signalwechselmenge.

6. Verfahren nach Anspruch 1, wobei es sich bei dem Datensatz des Schritts (a) um einen Rohdatensatz, einen mathematisch modifizierten Datensatz des Rohdatensatzes, einen normalisierten Datensatz des Rohdatensatzes oder einen normalisierten Datensatz des modifizierten Datensatzes des Rohdatensatzes handelt.

7. Verfahren nach Anspruch 1, wobei es sich bei dem Datensatz mit Signalwechselmenge um einen basislinienbereinigten Datensatz mit Signalwechselmenge handelt.

8. Verfahren nach Anspruch 1, wobei die Signalwechselmenge durch ein lineares Regressionsanalyseverfahren erlangt wird.

9. Verfahren nach Anspruch 1 und 8, wobei das Verfahren ferner den Schritt des Wiederholens der Schritte (b) bis (c) zum Glätten des Datensatzes umfasst, wobei der durch Schritt (c) bereitgestellte rekonstruierte Datensatz als Datensatz für Schritt (b) verwendet wird.

10. Computerlesbares Speichermedium, das Anweisungen enthält, um einen Prozessor so zu konfigurieren, dass er ein Verfahren zum Nachweis eines Zielanalyten in einer Probe ausführt, wobei das Verfahren Folgendes umfasst:
- (a) Empfangen eines Datensatzes für den Zielanalyten,
wobei der Datensatz aus einem Signalerzeugungsprozess für den Zielanalyten unter Verwendung eines Signalerzeugungsmittels erlangt wird, wobei der Datensatz eine Vielzahl von Datenpunkten umfasst, wobei jeder Datenpunkt einen Zyklus und einen Signalwert bei dem Zyklus umfasst;
- (b) Bereitstellen eines Datensatzes mit Signalwechselmenge, indem bei jedem Zyklus unter Verwendung der Signalwerte des Datensatzes eine Signalwechselmenge erlangt wird, wobei der Datensatz mit Signalwechselmenge eine Vielzahl von Datenpunkten umfasst, die die Zyklen und die Signalwechselmengen bei den Zyklen umfassen;
- (c) Bereitstellen eines rekonstruierten Datensatzes durch Erlangen eines kumulierten Werts bei jedem Zyklus unter Verwendung der Signalwechselmengen des Datensatzes mit Signalwechselmenge, wobei der rekonstruierte Datensatz eine Vielzahl von Datenpunkten umfasst, die Zyklen und kumulierte Werte bei dem Zyklus umfassen; und
- (d) Feststellen des Vorhandenseins oder Fehlens des Zielanalyten in der Probe unter Verwendung des rekonstruierten Datensatzes.

## Revendications

1. Procédé de détection d'un analyte cible dans un échantillon, comprenant :
- (a) la fourniture d'un ensemble de données pour l'analyte cible, l'ensemble de données étant obtenu à partir d'un processus de génération de signal pour l'analyte cible à l'aide d'un moyen de génération de signal, l'ensemble de données comprenant une pluralité de points de données, chaque point de données comprenant un cycle et une valeur de signal au cycle;
- (b) la fourniture d'un ensemble de données de quantité de changement de signal en obtenant une quantité de changement de signal pour chaque cycle à l'aide des valeurs de signal de l'ensemble de données, l'ensemble de données de quantité de changement de signal comprenant une pluralité de points de données comprenant les cycles et les quantités de changement de signal aux cycles;
- (c) la fourniture d'un ensemble de données reconstruit en obtenant une valeur cumulée à chaque cycle à partir des quantités de changement de signal de l'ensemble de données de quantité de changement de signal, l'ensemble de données reconstruit comprenant une pluralité de points de données comprenant des cycles et des valeurs cumulées aux cycles; et
- (d) la détection de l'analyte cible dans l'échantillon à l'aide de l'ensemble de données reconstruit.

2. Procédé selon la revendication 1, dans lequel la valeur cumulée à chaque cycle est calculée selon l'un des calculs suivants en fonction du numéro de chaque cycle (Xᵢ) par rapport au numéro d'un cycle de début de cumul (CSC), le cycle de début de cumul (CSC) étant un cycle sélectionné parmi les cycles de l'ensemble de données de quantité de changement de signal;
- (Cal-1) où, lorsque Xᵢ est supérieur au numéro de CSC, la valeur cumulée audit chaque cycle est calculée en cumulant (i) une valeur de début de cumul et (ii) une ou plusieurs quantités de changement de signal depuis un cycle suivant immédiatement le cycle de début de cumul jusqu' audit chaque cycle, la valeur de début de cumul étant une valeur cumulée au cycle de début de cumul;
- (Cal-2) où, lorsque Xᵢ est inférieur au numéro de CSC, la valeur cumulée audit chaque cycle est calculée en cumulant (i) la valeur de début de cumul et (ii) une ou plusieurs valeurs dérivées de la ou des quantités de changement de signal depuis un cycle suivant immédiatement ledit chaque cycle justqu' au cycle de début de cumul; et
- (Cal-3) où, lorsque Xᵢ est égal au numéro de CSC, la valeur de début de cumul est désignée comme la valeur cumulée audit chaque cycle.

3. Procédé selon la revendication 1, dans lequel la quantité de changement de signal à chaque cycle est choisie parmi le groupe constitué d'une valeur de dérivée des valeurs de signal à chaque cycle, d'une différence entre les valeurs de signal à chaque cycle, d'un rapport entre les valeurs de signal à chaque cycle et d'une valeur de pente obtenue en effectuant une analyse de régression linéaire à chaque cycle.

4. Procédé selon la revendication 1, dans lequel la valeur cumulée à chaque cycle est choisie parmi le groupe constitué d'une valeur cumulée des valeurs de dérivées à chaque cycle, d'une valeur cumulée des différences à chaque cycle, d'une valeur cumulée des rapports à chaque cycle et d'une valeur cumulée des valeurs de pente.

5. Procédé selon la revendication 1, lequel comprend en outre l'étape consistant à :
modifier au moins une ou plusieurs quantités de changement de signal de l'ensemble de données de quantité de changement de signal.

6. Procédé selon la revendication 1, dans lequel l'ensemble de données de l'étape (a) est un ensemble de données brutes, un ensemble de données modifié mathématiquement de l'ensemble de données brutes, un ensemble de données normalisé de l'ensemble de données brutes ou un ensemble de données normalisé de l'ensemble de données modifié de l'ensemble de données brutes.

7. Procédé selon la revendication 1, dans lequel l'ensemble de données de quantité de changement de signal est un ensemble de données de quantité de changement de signal après soustraction de la ligne de base.

8. Procédé selon la revendication 1, dans lequel la quantité de changement de signal est obtenue par une méthode d'analyse de régression linéaire.

9. Procédé selon les revendications 1 et 8, lequel comprend en outre l'étape consistant à répéter les étapes (b) à (c) pour lisser l'ensemble de données, l'ensemble de données reconstruit obtenu à l'étape (c) étant utilisé comme l'ensemble de données pour l'étape (b).

10. Support de stockage lisible par ordinateur contenant des instructions destinées à configurer un processeur pour qu'il mette en œuvre un procédé de détection d'un analyte cible dans un échantillon, le procédé comprenant :
- (a) la réception d'un ensemble de données pour l'analyte cible,
l'ensemble de données étant obtenu à partir d'un processus de génération de signal pour l'analyte cible à l'aide d'un moyen de génération de signal, l'ensemble de données comprenant une pluralité de points de données, chaque point de données comprenant un cycle et une valeur de signal au cycle;
- (b) la fourniture d'un ensemble de données de quantité de changement de signal en obtenant une quantité de changement de signal pour chaque cycle à l'aide des valeurs de signal de l'ensemble de données, l'ensemble de données de quantité de changement de signal comprenant une pluralité de points de données comprenant les cycles et les quantités de changement de signal aux cycles;
- (c) la fourniture d'un ensemble de données reconstruit en obtenant une valeur cumulée à chaque cycle à partir des quantités de changement de signal de l'ensemble de données de quantité de changement de signal, l'ensemble de données reconstruit comprenant une pluralité de points de données comprenant des cycles et des valeurs cumulées au cycle; et
- (d) la détermination de la présence ou de l'absence de l'analyte cible dans l'échantillon à l'aide de l'ensemble de données reconstruit.
